# EUROPEAN PATENT SPECIFICATION

(11) **EP 4 081 800 B1**
(45) Date of publication and mention of the grant of the patent: **27.05.2026**
(21) Application number: 20804551.8
(22) Date of filing: 13.11.2020
(51) Int. Cl.: G01N 33/574, G01N 33/50

(54) **METHOD FOR DIAGNOSING CUTANEOUS T-CELL LYMPHOMA DISEASES**
VERFAHREN ZUR DIAGNOSE KUTANER T-ZELL-LYMPHOMA ERKRANKUNGEN
PROCÉDÉ POUR LE DIAGNOSTIC DE MALADIES DE LYMPHOMES CUTANÉS À CELLULES T

(30) Priority: 27.12.2019 EP 19219889; 19.02.2020 EP 20158355; 12.05.2020 EP 20174174
(43) Date of publication of application: 02.11.2022
(73) Proprietor: Scailyte AG, 4056 Basel (CH); Universität Zürich, 8006 Zürich (CH)
(72) Inventor: NESTOROV, Peter, 6210 Sursee (CH); TIWARI, Vijay Kumar, 6210 Sursee (CH); CLAASSEN, Manfred, 6210 Sursee (CH); KNUCKLES, Philip, 6210 Sursee (CH); HAMILTON CARL, Sarah, 6210 Sursee (CH); GUENOVA, Emmanuella, 8091 Zürich (CH); FASSNACHT, Christina, 8091 Zürich (CH); VARYPATAKI, Eleni Maria, 8091 Zürich (CH)
(74) Representative: Vossius & Partner Patentanwälte Rechtsanwälte mbB
(86) International application number: PCT/EP2020/082068
(87) International publication number: WO 2021/129976

(56) References cited:
- WO-A1-2010/140885
- WO-A1-2013/071410
- CHRIS H JOKINEN ET AL: "Flow Cytometric Evaluation of Skin Biopsies for Mycosis Fungoides :", AMERICAN JOURNAL OF DERMATOPATHOLOGY, vol. 33, no. 5, 1 July 2011 (2011-07-01), US, pages 483 - 491, XP055716743, ISSN: 0193-1091, DOI: 10.1097/DAD.0b013e31820595da
- WASHINGTON LABARON T ET AL: "A stable aberrant immunophenotype characterizes nearly all cases of cutaneous T-cell lymphoma in blood and can be used to monitor response to therapy", BMC CLINICAL PATHOLOGY, BIOMED CENTRAL, LONDON, GB, vol. 2, no. 1, 10 December 2002 (2002-12-10), pages 5, XP021017372, ISSN: 1472-6890, DOI: 10.1186/1472-6890-2-5
- PEDRO HORNA ET AL: "Single-Antibody Detection of T-Cell Receptor Beta Chain Monotypia Resolves Uncertainties in the Identification and Quantitation of S�zary Cells By Routine Flow Cytometry: Towards Accurate and Unequivocal Blood Staging of Cutaneous T-Cell Lymphomas", BLOOD 134 (S1), PAGE 2848, 13 November 2019 (2019-11-13), XP055716740, Retrieved from the Internet <URL:https://ashpublications.org/blood/article/134/Supplement_1/2848/423477/Single-Antibody-Detection-of-T-Cell-Receptor-Beta> [retrieved on 20200721]
- ANONYMOUS: "Phenopath Pathology Reference Guide", 1 October 2016 (2016-10-01), XP055716727, Retrieved from the Internet <URL:http://phenopath.com/uploads/pdf/pathology-reference-guide-2016.pdf> [retrieved on 20200721]
- MURRAY DUNCAN ET AL: "'T-cell versus T-cell': Tumour infiltrating lymphocytes in mycosis fungoides show a remarkably homogeneous exhaustion profile across a heterogeneous patient population", EUROPEAN JOURNAL OF CANCER, ELSEVIER, AMSTERDAM NL, vol. 101, 28 August 2018 (2018-08-28), XP085456882, ISSN: 0959-8049, DOI: 10.1016/J.EJCA.2018.07.172
- JARIWALA NEHA: "TIGIT and Helios Are Highly Expressed on CD4D T Cells in Sezary Syndrome Patients", JOURNAL OF INVESTIGATIVE DERMATOLOGY, 20 October 2016 (2016-10-20), XP055771939, Retrieved from the Internet <URL:https://api.elsevier.com/content/article/PII:S0022202X16322503?httpAccept=text/plain> [retrieved on 20210204]
- FLORIAN ANZENGRUBER ET AL: "Divergent LAG-3 versus BTLA, TIGIT, and FCRL3 expression in Sézary syndrome", LEUKEMIA AND LYMPHOMA., vol. 60, no. 8, 14 January 2019 (2019-01-14), US, pages 1899 - 1907, XP055771950, ISSN: 1042-8194, DOI: 10.1080/10428194.2018.1564827
- ANZENGRUBER FLORIAN ET AL: "Expression of TIGIT, BTLA, LAG3, FCRL3 and CTLA-4 in Sézary Syndrome", EUROPEAN JOURNAL OF CANCER, ELSEVIER, AMSTERDAM NL, vol. 101, 28 August 2018 (2018-08-28), XP085456863, ISSN: 0959-8049, DOI: 10.1016/J.EJCA.2018.07.153
- DUNCAN MURRAY ET AL: "Progression of mycosis fungoides occurs through divergence of tumorimmunophenotype by differential expression of HLA-DR", BLOOD ADVANCES, vol. 3, no. 4, 26 February 2019 (2019-02-26), pages 519 - 530, XP055771966, ISSN: 2473-9529, DOI: 10.1182/bloodadvances.2018025114
- K. STASER ET AL: "Sézary syndrome patient-derived xenografts for 21-color flow cytometry immunophenotyping and CART cell therapeutic testing", JOURNAL OF INVESTIGATIVE DERMATOLOGY, vol. 138, no. 5, 19 April 2018 (2018-04-19), NL, pages S75, XP055771976, ISSN: 0022-202X, DOI: 10.1016/j.jid.2018.03.448

## Description

The invention relates to biomarkers useful for the diagnosis of cutaneous T-cell lymphoma (CTCL) diseases, in particular Sézary syndrome and/or mycosis fungoides, and/or for the differential diagnosis of Sézary syndrome and/or mycosis fungoides as opposed to atopic dermatitis and other medical conditions. The biomarkers provided herein are also useful for monitoring treatment of the disease, relapse of the disease and/or as targets for therapeutic intervention. In particular, the invention relates to a method for determining the frequency of Sézary signature cells and/or mycosis fungoides cells in a plurality of cells, a computer-implemented method for determining the frequency of Sézary signature cells or mycosis fungoides cells, a method for diagnosing a subject as having Sézary syndrome or as having mycosis fungoides, a method for determining the susceptibility to treatment of Sézary syndrome or mycosis fungoides in a subject, and a composition comprising reagents for the detection of biomarkers for the diagnosis of Sézary syndrome or mycosis fungoides.

Cutaneous T cell lymphomas (CTCLs) are a group of T cell neoplasms with skin involvement. The two most prevalent types of CTCL are mycosis fungoides (MF) and Sézary syndrome (SS), both of which are thought to be derived from mature skin-homing CD4+ T cells. SS refers to a rare form of CTCL characterized by circulating malignant cells with widespread skin involvement and a 20%survival rate over 5 years after. Prognosis for SS is challenging, partially due to an incomplete understanding of disease pathogenesis. At advanced stages, CTCL causes significant damage to the patient's immune system and a severe skin inflammation that covers >80% of the body.

Unambiguous diagnosis of Sézary syndrome or mycosis fungoides in its initial stages is currently severely hampered in clinical practice by the occurrence of other diseases with similar symptoms such as psoriasis, atopic dermatitis (AD), chronic eczema and other forms of non-specific dermatitis. Patients with CTCL are often misdiagnosed with AD which is a highly prevalent skin condition that has similar clinical presentation and histological profile. This combined with a lack of a definitive diagnostic test for CTCLs means that this disease is typically diagnosed in the advanced stages. Delayed diagnosis results in incorrect treatment strategies, morbidity, reduced quality of life (QoL) and a significant economic burden.

Mycosis fungoides is the most common form of cutaneous T-cell lymphoma. It generally affects the skin, but may progress into the blood over time. The cause of mycosis fungoides is unknown, but it is not believed to be hereditary or genetic in the vast majority of cases. It is not contagious, although some research suggests that the Human T-lymphotropic virus is associated with this condition. The disease is an unusual expression of CD4+ T cells. These T-cells are skin-associated, meaning they are biochemically and biologically most related to the skin, in a dynamic manner. Mycosis fungoides is the most common type of cutaneous T-cell lymphoma (CTCL), but there are many other types of CTCL that have nothing to do with mycosis fungoides and these disorders are treated differently.

Diagnosis of CTCL is extremely challenging and requires multiple technologies. Presently the accepted WHO-EORTC classification is used for diagnosis and is based on a combination of clinical and histopathological markers. These include assays based on Histology, Molecular Biology, White blood cell morphology and quantification. In addition, clinicians may rely on flow cytometry for blood analysis and whole-body imaging tests (e.g. CT scan, PET scan, MRI) for determining the severity of the disease.

The Wistar Institute has also developed a test to aid in the diagnosis of patients with CTCLs Li J et al. Cancer Manag Res. 2012 Mar 12;4:75-89. This test is based on the use of complementary DNA (cDNA) microarray technology to identify a specific gene expression profile in patient peripheral blood mononuclear cells (PBMCs). However, such technology cannot measure expression on a single-cell level, but is rather restricted to analyzing a bulk, averaged samples, thereby reducing its sensitivity.

WO2010140885 provides panels of antibody reagents conjugated to fluorescent compounds for the identification and characterization of a leukocyte population of interest. Further, disclosed is a flow cytometric panel for identification of Sézary syndrome using the biomarkers CD2, CD3, CD4, CD7, CD8/CD8a, CD26, CD28 and CD45.

Pedro Honra, et al. (2019) Blood 134 (S1): 2848 added TRBC1 to a routine Sézary cell flow cytometry panel using CD2, CD3, CD4, CD5, CD7, CD8, CD26, CD45 to study 33 peripheral blood specimens from 24 patients with cutaneous T-cell lymphoma, and 28 specimens from patients with no clinical or laboratory evidence of T-cell malignancy. With this 9-biomarker panel Honra et al. identified 24 of 33 specimens of patients with cutaneous T-cell lymphoma.

Washington, LaBaron et al. (2002) BMC clinical pathology Vol. 2(1): 5, describes the incidence of aberrancies in commonly expressed T-cell markers in tumor cells of mycosis fungoides and Sézary syndrome in a flow cytometric panel using the markers CD3, CD4, CD5, CD7, CD8/CD8a, CD26, CD45 and TCR to monitor response to therapy.

Jokinen et al. (2011) The American Journal of dermatopathology 33(5): 483-91 describes a flow cytometric evaluation of skin biopsies for diagnosis and classification of mycosis fungoides using a panel with the biomarkers CD2, CD3, CD4, CD5, CD7, CD8/CD8a, CD45 and CD56.

WO 2013/071410 A1 discloses biomarkers including TOX useful for the screening for, diagnosing or detecting of T cell malignancy. A level of one or more biomarkers including TOX is determined in a sample from the subject and compared to a control level, wherein an increased level of the one or more biomarkers in the sample relative to the control level indicates that the subject has T cell malignancy.

In the study of Murray et al., 2018 (EUROPEAN JOURNAL OF CANCER, ELSEVIER, AMSTERDAM NL, vol. 101, 28 August 2018, DOI 10.1182/bloodadvances.2018025114), the authors sought to investigate TIL phenotype by using TCRVβ-based flow cytometric clonotyping to differentiate tumor and TIL T-cell subsets. Expression of additional markers such as PD-1, PD-L1, Fas, HLA-DR. TIGIT and granzyme B was performed. Skin TILs from mycosis fungoides patients showed marked upregulation of immune checkpoint receptors PD-1 and TIGIT compared to peripheral blood and healthy donors. Fas, MHC-I and HLA-DR were also highly expressed.

In the study of Jariwala Neha (Journal of Investigative Dermatology, 20 October 2016) high expression of FCRL3 on CD26- CD4 + T cells in Sézary Syndrome (SS) patients with high blood tumor burden are reported. In addition, increased percentages of TIGIT 4- and Helios+ CD4 + T cells were detected in patients with SS when compared with patients with mycosis fungoides patients and healthy donors.

Anzengruber et al., 2019 (LEUKEMIA AND LYMPHOMA, vol. 60, no. 8, 14 January 2019, DOI: 10.1080/10428194.2018.1564827) investigated expression of BTLA, CTLA-4, FCRL3, LAG-3, and TIGIT in tumor and non-tumor Sézary syndrome (SS) T-cells was evaluated. Compared to CD4+ T helper cells from ten healthy individuals, tumor cells of eight SS patients showed a significant upregulation of BTLA, FRCL3 and TIGIT expression. In contrast, a reduced expression of LAG-3+ cells was found in the blood of tumor patients.

Anzengruber et al., 2018, (EUROPEAN JOURNAL OF CANCER, ELSEVIER, AMSTERDAM NL, vol. 101, 28 August 2018, DOI: 10.1016/J.EJCA.2018.07.153) evaluated the expression of BTLA, TIGIT, LAG-3, and FCRL3 in a cohort of Sézary syndrome (SS) patients with an identifiable Vβ clone. Expression of TIGIT and BTLA was increased, while the percentage of LAG-3 expressing cells was reduced in SS-tumor T-cells compared to T-cells from healthy individuals.

Murray et al., 2019 (BLOOD ADVANCES, vol. 3, no. 4, 26 February 2019, DOI: 10. 1182/bloodadvances.2018025114) T-cell receptor (TCR)-based identification of the tumor population was used to undertake a phenotypic and functional analysis of the CD4+ and CD8+ TILs and contrast this with features of the malignant cells. TILs were demonstrated to express a homogeneous phenotype and function across all patient groups, whereas tumor cells are markedly heterogeneous between different patients.

Staser et al., 2018, (JOURNAL OF INVESTIGATIVE DERMATOLOGY, vol. 138, no. 5, 19 April 2018, DOI: 10.1016/j.jid.2O18.O3.448) generated Sézary syndrome (SS) patient-derived xenografts (PDXs) to characterize immunophenotypes and test off-the-shelf universal chimeric antigen receptor T (UCART) cells. Pre-engraftment SS cells were predominantly CD3+/4+/5+/2++ with variable CD7 loss and PD1 gain. Engrafted cells showed near ubiquitous PD1 expression and CD7 loss.

Thus, there is still a need for methods to reliably diagnose Sézary syndrome and/or mycosis fungoides in a subject with high sensitivity and specificity.

The above technical problem is solved by the present invention as defined in the claims.

The inventors have identified a set of cell-surface proteins, and/or biomarkers, which in combination, define a unique sub-population of immune cells. The presence of these cells, herein referred to as Sézary signature cells or mycosis fungoides cells in PBMC samples, as determined using e.g. an antibody-based assay such as flow or mass cytometry, is able to separate a subject with Sézary syndrome or mycosis fungoides, respectively, from a healthy individual or a subject with atopic dermatitis or other medical indications with a high sensitivity and specificity. Sézary signature cells and mycosis fungoides cells express either a higher or a lower level of a plurality of the given cell-surface proteins than other immune cell types that can be identified in PBMC samples. These Sézary signature cells or mycosis fungoides cells are present at a higher frequency in a subject with Sézary syndrome or mycosis fungoides, respectively, than in a healthy individual and/or a subject with atopic dermatitis or other medical conditions.

Detection of the presence or absence of these Sézary signature cells or mycosis fungoides cells, and/or of changes in their levels over time can thus be used to indicate that a subject has Sézary syndrome or mycosis fungoides, respectively. In addition, the method of the present invention may be used to monitor the progression of Sézary syndrome or mycosis fungoides over time or after treatment.

That is, the invention relates to a method for determining the frequency of Sézary signature cells and/or mycosis fungoides cells in a plurality of cells, the method comprising the steps of:
i) determining the levels of expression of 9 or more biomarkers in a plurality of cells; and
ii) determining the frequency of Sézary signature cells and/or mycosis fungoides cells in the plurality of cells based on the levels of expression of the 9 or more, biomarkers; wherein the biomarkers comprise or consist of
   a) CD28, CD44, HLA-ABC, CD26, CD3, CD195, CD164, CD8/CD8a, HLA-DR, CD194, CD11b, CD38, CD45, CD95, CD223, CD45RA, CD14, CD7, CD123, TIM3, TIGIT, CD19, and FcRL3,
   b) CD28, PD-1, CD4, CD3, HLA-ABC, TIGIT, CD197, CD27, CD26, CD44, CD194, CD7, CD45RA, CD158k, and CD164,
   c) CD3, CD4, CD7, CD8/CD8a, CD26, CD27, CD45, CD45RA and TIGIT, or
   d) CD3, CD4, CD7, CD8/CD8a, CD26, CD45RO, TIGIT, CD194/CCR4, CD197/CCR7, and CD279/PD-1.

Preferably, a classifier algorithm is used to distinguish between a Sézary signature cell and a non-Sézary signature cell or a mycosis fungoides cell and a non-mycosis fungoides cell, respectively.

Preferably, a convolutional neural network is used to distinguish between a Sézary signature cell and a non-Sézary signature cell or a mycosis fungoides cell and a non- mycosis fungoides cell, respectively.

Preferably, the plurality of cells are human cells.

Preferably, the levels of the biomarkers are determined using an antibody-based assay.

Preferably, the antibody-based assay is an antibody-based flow cytometry or mass cytometry assay.

Preferably, the biomarkers are:
r) a panel comprising or consisting of nine different biomarkers, namely (i) CD3, (ii) CD4, (iii) CD7, (iv) CD8/CD8a, (v) CD26, (vi) CD27, (vii) CD45, (viii) CD45RA and (ix) TIGIT;
u) a panel comprising or consisting of ten different biomarkers, namely (i) CD3, (ii) CD4, (iii) CD7, (iv) CD8/CD8a, (v) CD26, (vi) CD45RO, (vii) TIGIT, (viii) CD194, (ix) CD197 and (x) PD-1.

Preferably, the 9 or more biomarkers are a panel comprising or consisting of nine different biomarkers, namely (i) CD3, (ii) CD4, (iii) CD7, (iv) CD8/CD8a, (v) CD26, (vi) CD27, (vii) CD45, (viii) CD45RA and (ix) TIGIT.

The invention further relates to a computer-implemented method for determining the frequency of Sézary signature cells or mycosis fungoides cells, the method comprising the steps of:
i) executing a classifier algorithm on a set of data comprising the levels of expression of 9 or more biomarkers in a plurality of cells;
ii) determining the frequency of Sézary signature cells or mycosis fungoides cells, respectively, in the plurality of cells underlying the set of data based on the levels of expression of the 9 or more biomarkers, wherein the biomarkers comprise or consist of
   a) CD28, CD44, HLA_ABC, CD26, CD3, CD195, CD164, CD8/CD8a, HLA-DR, CD194, CD11b, CD38, CD45, CD95, CD223, CD45RA, CD14, CD7, CD123, TIM3, TIGIT, CD19, and FcRL3,
   b) CD28, PD-1, CD4, CD3, HLA ABC, TIGIT, CD197, CD27, CD26, CD44, CD194, CD7, CD45RA, CD158k, and CD164,
   c) CD3, CD4, CD7, CD8/CD8a, CD26, CD27, CD45, CD45RA and TIGIT, or
   d) CD3, CD4, CD7, CD8/CD8a, CD26, CD45RO, TIGIT, CD194/CCR4, CD197/CCR7, and CD279/PD-1.

Preferably, the classifier algorithm comprises one or a combination of a support vector algorithm, a convolutional neural network, a tree-based method, logistic regression.

The disclosure further relates to a computer program product containing instructions for performing the computer-implemented method according to the invention.

The invention further relates to a method for diagnosing a subject as having Sézary syndrome or as having mycosis fungoides, the method comprising the steps of:
i) determining the frequency of Sézary signature cells or mycosis fungoides cells, respectively, in a sample obtained from the subject using the method according to the invention;
ii) comparing the frequency of Sézary signature cells or mycosis fungoides cells, respectively, determined in step (i) to the frequency of Sézary signature cells or mycosis fungoides cells, respectively, in a sample that has been obtained from a subject not suffering from Sézary syndrome or mycosis fungoides, respectively, and/or to the frequency of Sézary signature cells or mycosis fungoides cells, respectively, in a sample that has been obtained from a subject with Sézary syndrome or mycosis fungoides, respectively; and
iii) determining a subject as having Sézary syndrome or mycosis fungoides, respectively, if the frequency of Sézary signature cells or mycosis fungoides cells, respectively, determined in step (ii) is higher compared to the frequency of Sézary signature cells or mycosis fungoides cells, respectively, for the subject not suffering from Sézary syndrome or mycosis fungoides, respectively, and/or if the frequency of Sézary signature cells or mycosis fungoides cells, respectively, determined in step (ii) is similar or higher compared to the frequency of Sézary signature cells or mycosis fungoides cells, respectively, in the sample that has been obtained from the subject with Sézary syndrome or mycosis fungoides, respectively.

Preferably, determining the subject as having Sézary syndrome or mycosis fungoides, respectively, comprises the use of a classifier algorithm.

Preferably, the classifier algorithm comprises a convolutional neural network and/or logistic regression.

Preferably, the subject without Sézary syndrome is a healthy subject or a subject suffering from atopic dermatitis, non-specific dermatitis, erythroderma and/or mycosis fungoides.

Preferably, the method for diagnosing a subject as having Sézary syndrome or as having mycosis fungoides further comprising one or more other diagnostic tests and/or additional information about the subject.

Preferably, the one or more other diagnostic tests is/are selected from the group consisting of: whole-body imaging tests, skin lesion biopsies, histopathology tests, CD4/CD8 ratio determination, cell count analysis and PCR analysis of T cell receptor clonality.

Preferably, the additional information about the subject comprises age and/or clinical information.

The invention further relates to a method for determining the susceptibility to treatment of Sézary syndrome or mycosis fungoides in a subject, the method comprising the steps of:
i) determining the frequency of Sézary signature cells or mycosis fungoides cells, respectively, in a first sample that has been obtained from said subject;
ii) determining the frequency of Sézary signature cells or mycosis fungoides cells, respectively, in a second sample that has been obtained from the same subject, wherein the second sample has been obtained at a later time point than the first sample;
iii) determining the change in frequency of Sézary signature cells or mycosis fungoides cells, respectively, between the first and second sample; and iv) determining the subject as being susceptible to treatment of Sézary syndrome or mycosis fungoides, respectively, if the frequency of Sézary signature cells or mycosis fungoides cells, respectively, is lower in the second sample than in the first sample;
wherein the frequency of Sézary signature cells or mycosis fungoides cells, respectively, is determined using the method according to the invention.

Preferably, the sample is a blood sample, in particular, wherein the sample comprises peripheral blood mononuclear cells.

The invention further relates to a composition consisting of reagents for the detection of biomarkers for the diagnosis of Sézary syndrome or mycosis fungoides, the biomarkers consisting of:
a) CD28, CD44, HLA_ABC, CD26, CD3, CD195, CD164, CD8/CD8a, HLA-DR, CD194, CD11b, CD38, CD45, CD95, CD223, CD45RA, CD14, CD7, CD123, TIM3, TIGIT, CD19, and FcRL3,
b) CD28, PD-1, CD4, CD3, HLA ABC, TIGIT, CD197, CD27, CD26, CD44, CD194, CD7, CD45RA, CD158k, and CD164,
c) CD3, CD4, CD7, CD8/CD8a, CD26, CD27, CD45, CD45RA and TIGIT, or
d) CD3, CD4, CD7, CD8/CD8a, CD26, CD45RO, TIGIT, CD194/CCR4, CD197/CCR7, and CD279/PD-1.

Preferably, the composition comprises reagents for the detection of biomarkers for the diagnosis of Sézary syndrome or mycosis fungoides, the biomarkers consisting of:
a) CD3, CD4, CD7, CD8/CD8a, CD26, CD27, CD45, CD45RA and TIGIT; or
b) CD3, CD4, CD7, CD8/CD8a, CD26, CD45RO, TIGIT, CD194, CD197 and PD-1.

Preferably, the composition comprises reagents for the detection of biomarkers for the diagnosis of Sézary syndrome or mycosis fungoides, the biomarkers consisting of CD3, CD4, CD7, CD8/CD8a, CD26, CD27, CD45, CD45RA and TIGIT.

Accordingly, in one embodiment, the invention relates to a method for determining the frequency of Sézary signature cells and/or mycosis fungoides cells in a plurality of cells, the method comprising the steps of:
i) determining the levels of expression of 9 or more biomarkers in a plurality of cells; and
ii) determining the frequency of Sézary signature cells and/or mycosis fungoides cells in the plurality of cells based on the levels of expression of the 9 or more, biomarkers;
wherein the biomarkers comprise or consist of
a) CD28, CD44, HLA-ABC, CD26, CD3, CD195, CD164, CD8/CD8a, HLA-DR, CD194, CD11b, CD38, CD45, CD95, CD223, CD45RA, CD14, CD7, CD123, TIM3, TIGIT, CD19, and FcRL3,
b) CD28, PD-1, CD4, CD3, HLA-ABC, TIGIT, CD197, CD27, CD26, CD44, CD194, CD7, CD45RA, CD158k, and CD164,
c) CD3, CD4, CD7, CD8/CD8a, CD26, CD27, CD45, CD45RA and TIGIT, or
d) CD3, CD4, CD7, CD8/CD8a, CD26, CD45RO, TIGIT, CD194/CCR4, CD197/CCR7, and CD279/PD-1.

**Table 1**

| **Target** |
|---|
| CD45 |
| CD196/CCR6 |
| CD19 |
| CD307c/FcRL3 |
| HLA-ABC |
| CD4 |
| CD8/CD8a |
| CD7 |
| CD14 |
| CD25 (IL-2R) |
| CD61 |
| CD123 |
| CD95 [Fas] |
| CD366 [Tim3] |
| TIGIT |
| CD279/PD-1 |
| CD195/CCR5 |
| CD194/CCR4 |
| CD 197/CCR7 |
| CD28 |
| CD26 |
| CD11c |
| CD164 |
| CCL17/CADM1 |
| CD16 |
| CD44 |
| CD27 |
| CD127/IL-7Ra |
| CD45RA |
| CD3 |
| CD20 |
| CD38 |
| KIR3DL2/CD158k |
| HLA-DR |
| CD223 [LAG3] |
| CD56 |
| CD45RO |

The present invention relates to a method for the detection of Sézary syndrome and/or mycosis fungoides with high specificity and sensitivity. Other approaches for the detection of Sézary syndrome, e.g. the approach cited above, were based on determining the average expression of marker genes in a cell population or were using a suboptimal combination of biomarkers and therefore suffered from reduced sensitivity. The method of the present invention, on the other hand, allows measuring the expression level of biomarkers at the single-cell level. That is, for each cell in a plurality of cells, the expression level of 9 or more biomarkers is determined and based on the expression level of these biomarkers, it is decided for each cell if it is indicative of at least one, preferably more, medical condition(s), such as Sézary syndrome and/or mycosis fungoides. The frequency of these indicative cells in a plurality of cells may then be used for determining if a subject from which the plurality of cells has been obtained, the donor, suffers from a certain medical condition and/or for determining the progression of a certain medical condition in a subject from which the plurality of cells has been obtained. The panels provided in the present invention comprise 9 or more biomarkers selected from
a) CD28, CD44, HLA-ABC, CD26, CD3, CD195, CD164, CD8/CD8a, HLA-DR, CD194, CD11b, CD38, CD45, CD95, CD223, CD45RA, CD14, CD7, CD123, TIM3, TIGIT, CD19, and FcRL3, b) CD28, PD-1, CD4, CD3, HLA-ABC, TIGIT, CD197, CD27, CD26, CD44, CD194, CD7, CD45RA, CD158k, and CD164, c) CD3, CD4, CD7, CD8/CD8a, CD26, CD27, CD45, CD45RA and TIGIT, or d) CD3, CD4, CD7, CD8/CD8a, CD26, CD45RO, TIGIT, CD194/CCR4, CD197/CCR7, and CD279/PD-1. In particular the panels comprising biomarkers selected from the group consisting CD3, CD4, CD7, CD8/CD8a, CD26, CD27, CD45, CD45RA and TIGIT result in a more sensitive detection of Sézary cells in samples obtained from patients (Table 5, Table 8).

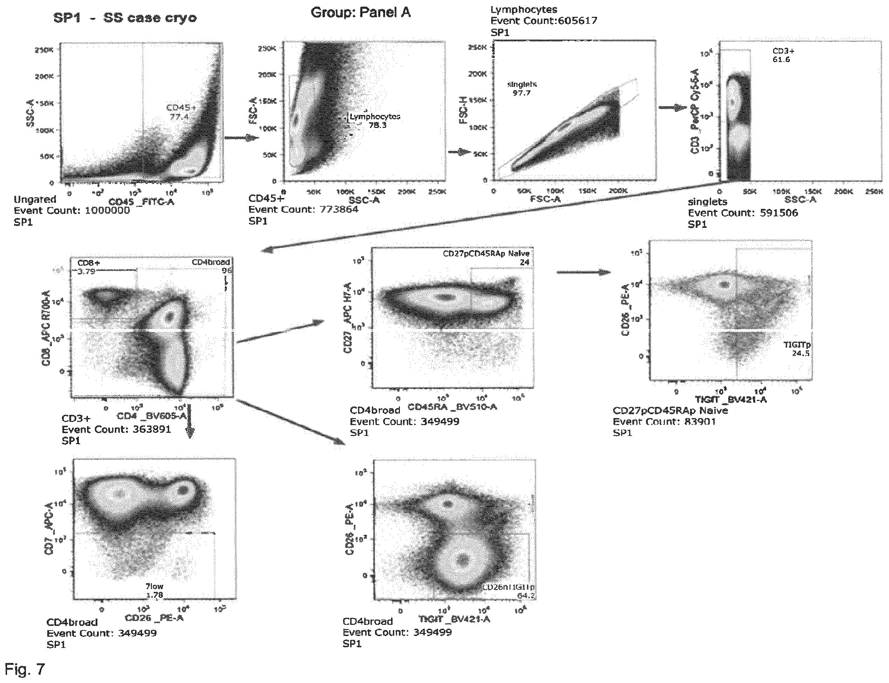

Therefore, determining the expression of the biomarker TIGIT and a certain number of biomarkers selected from the group listed in Table 1 is surprisingly useful in the diagnosis of Sézary syndrome and/or mycosis fungoides (Table 3, Table 4, Table 5, Table 8). Furthermore, the inventors demonstrated that certain combinations of some of the biomarkers listed in Table 1 are particularly useful for diagnosing Sézary syndrome and/or a mycosis fungoides (see, e.g., FIG. 7, Table 8). A method, wherein the levels of CD3, CD4, CD7, CD8/CD8a, CD26, CD27, CD45, CD45RA and/or TIGIT are determined in a plurality of cells enables the detection of Sézary syndrome and/or mycosis fungoides in a subject with high specificity and sensitivity (Table 8). While certain biomarkers (e.g., CD3, CD4, CD8/CD8a, and/or CD45) are useful for the initial separation of cells, other biomarkers (e.g., CD27, CD26, CD7, CD45RA and/or TIGIT) are useful for the specialized (e.g., more specific for Sézary syndrome and/or mycosis fungoides) separation of cells (Figure 7).

Therefore, using a panel comprising a certain number of the biomarkers according to Table 7, in particular biomarkers selected from the group of CD3, CD4, CD7, CD8/CD8a, CD26, CD27, CD45, CD45RA and TIGIT, preferably the biomarkers selected from the group of CD27, CD26, CD7, CD45RA and TIGIT is useful for the diagnosis of Sézary syndrome and/or mycosis fungoides.

The term "biomarker", as used herein, refers to a molecule that is part of and/or generated by a cell and serves as an indicator for a disease. Often a biomarker is a gene variant or a gene product, for example an RNA or a polypeptide. Within the present invention, the biomarkers are preferably proteins, preferably proteins that are localized on the surface of the cell, such that they are accessible to binding agents that can be used for the quantification of the biomarker. A biomarker may be any protein that is expressed on the surface of a cell. In certain embodiments, a biomarker is a cell surface protein that is known to be present on immune cells, such as CD4+ cells and/or a cell surface protein that is known or suspected to be associated to certain diseases, such as cancer and, in particular, cutaneous T cell lymphomas (CTCL).

Within the present disclosure the expression level of any number of biomarkers may be determined. The expression levels of the biomarkers that are listed in Table 1, or a subset thereof, may be measured in order to determine if a subject suffers from a certain medical condition, such as forms of CTCL, in particular Sézary syndrome or mycosis fungoides. That is the expression levels of all biomarkers from Table 1 may be determined in a cell in order to determine if said cell is indicative of a certain medical condition. The expression levels of only a subset of the biomarkers in Table 1 may be determined in a cell in order to determine if said cell is indicative of a certain medical condition. The expression levels of all biomarkers in Table 1, or of a subset of the biomarkers in Table 1 may be determined in a cell together with the expression level of other biomarkers that are not listed in Table 1 in order to determine if said cell is indicative of a certain medical condition.

The terms "expression level" or "level of expression", as used herein, refer to the absolute frequency/abundance of a biomarker described herein or the relative frequency/abundance as compared to a reference, in particular a known frequency/abundance on a healthy cell or a diseased cell to which the determined frequency/abundance can be compared. The expression level of a biomarker in a single cell may be measured by any method known in the art. The expression level of a biomarker may be measured on the nucleic acid level or on the protein level. That is, in certain embodiments, the expression level of a biomarker in a cell may be measured by determining the levels of mRNAs in said cell by methods known in the art, such as sequencing and/or PCR-based methods. In other embodiments, the expression level of a biomarker may be measured by determining the level of a protein in or on the surface of said cell. Measuring the level of a protein may comprise the use of a binding agent, such as an antibody that specifically binds to a target protein. The skilled person is aware of methods to determine the expression level of a protein in a single cell. Preferably, flow cytometry or mass cytometry methods known in the art may be used for determining the expression level of a protein in a single cell. Within the present invention, it is preferred that the binding agent binds to proteins on the cell surface. However, the present invention also encompasses embodiments wherein the biomarkers are intracellular biomarkers. In this case, cells may be fixated and/or permeabilized before addition of the binding agent.

The term "flow cytometry", as used herein, refers to methods for the analysis of cells or particulate samples well known to the skilled artisan, such as those provided by Becton-Dickinson, Cytomation, Partec, Luminex, or Beckman-Coulter. Flow cytometry can encompass multiparametric DNA analysis, platelet studies, reticulocyte enumeration, cell biology/functional studies, innovative research in immunobiology, cell physiology, molecular biology, genetics, microbiology, water quality and plant cell analysis as well as a broad range of research applications. Current flow cytometers are manufactured with the ability to measure more than one, three or more, preferably four or more separate detectable labels simultaneously. Using methods for flow cytometric analysis, a specifically labeled molecule, such as an antibody, is added to the cellular or particulate sample believed to contain an analyte of interest. The antibody is labeled with an appropriate detectable label, such as a fluorophore, which permits detection of those cells or particles comprising the analyte of interest at a detectable level. The analysis can involve quantification and/or detection of the analyte, and may also involve sorting or harvesting the cells or particles possessing the analyte of interest.

Mass cytometry is a mass spectrometry technique based on inductively coupled plasma mass spectrometry and time of flight mass spectrometry used for the determination of the properties of cells (cytometry). In this approach, antibodies are conjugated with isotopically pure elements, and these antibodies are used to label cellular proteins. Cells are nebulized and sent through an argon plasma, which ionizes the metal-conjugated antibodies. The metal signals are then analyzed by a time-of-flight mass spectrometer. The approach overcomes limitations of spectral overlap in flow cytometry by utilizing discrete isotopes as a reporter system instead of traditional fluorophores which have broad emission spectra.

The number of biomarkers that are analyzed in a cell may be dependent on the experimental method used. That is, the number of biomarkers may be dependent on the number of labels that can be simultaneously detected by a flow cytometer or on the number of metal-conjugated antibodies that are available for mass cytometry applications.

By measuring the expression of biomarkers in each cell in a plurality of cells, it is possible to determine the frequency of cells that are indicative of a certain medical condition. The "relative frequency" of an event is defined as the number of times that the event occurs during experimental trials, divided by the total number of trials conducted. That is, the relative frequency of cells that are indicative of a certain medical condition, such as a Sézary signature cells or a mycosis fungoides cells, in a plurality of cells may be determined by dividing the number of cells that are indicative of a certain medical condition by the total number of cells in a plurality of cells. A "plurality of cells", as used herein, is defined as two or more than two cells.

In certain embodiments, the method of the invention may be used for determining the relative frequency of Sézary signature cells in a plurality of cells.

Within the present invention, a "Sézary signature cell" is a cell that is indicative of Sézary syndrome. That is, if a certain relative frequency of Sézary signature cells is determined in a plurality of cells that has been obtained from a subject, the subject is diagnosed as having Sézary syndrome. Further, determining the relative frequency of Sézary signature cells in samples from the same subject at two or more time points allows to monitor the progression of Sézary syndrome in said subject.

The decision if a cell is a Sézary signature cell is based on the expression level of biomarkers in said cell. In certain embodiments, the set of biomarkers that is used for making the decision if a cell is a Sézary signature cell comprises 9, 10, 11, 12, 13, 14, 15, 16, 17, 18, 19, 20, 21, 22, 23, 24, 25, 26, 27, 28, 29, 30, 31, 32, 33, 34, 35 or 36 biomarkers. In other embodiments, the set of biomarkers that are used for the decision if a cell is a Sézary signature cell comprises further biomarkers that are not listed in Table 1.

Further disclosed is a method wherein two or more, three or more, preferably four or more, more preferably six or more, more preferably eight or more, more preferably nine or more, biomarkers are selected from the group of cell surface markers consisting of: CD28, CD197, CD3, HLA-ABC, CD194, CD26, CD164, CD44, CD45, CD45RA, CD45RO, CD7, CD4, CD27, TIGIT, PD-1, HLA-DR, CD14, CD158K, CD95, CD8/CD8a and CD195.

Further disclosed is a method, wherein the two or more, three or more, preferably four or more, more preferably six or more, more preferably seven or more biomarkers include TIGIT and further biomarkers are selected from the group of cell surface markers consisting of: CD28, CD197, CD3, HLA-ABC, CD194, CD26, CD164, CD44, CD45RA, CD7, CD4, CD27, PD-1, HLA-DR, CD14, CD158K, CD95, CD8/CD8a and CD195.

The biomarkers are preferably cell surface markers. A "cell surface marker", as used herein, is a protein that is expressed on the surface of a cell. Further disclosed are biomarkers selected from a group consisting of the cell surface markers CD28, CD197, CD3, HLA-ABC, CD194, CD26, CD164, CD44, CD45, CD45RA, CD45RO, CD7, CD4, CD27, TIGIT, PD-1, HLA-DR, CD14, CD158K, CD95, CD8/CD8a and CD195. That is, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19 or all 20 of the biomarkers CD28, CD197, CD3, HLA-ABC, CD194, CD26, CD164, CD44, CD45, CD45RA, CD45RO, CD7, CD4, CD27, TIGIT, PD-1, HLA-DR, CD14, CD158K, CD95, CD8/CD8a and CD195 may be used for the decision if a cell is a Sézary signature cell.

In some embodiments, the disclosure encompasses any possible combination of the biomarkers listed above in particular any possible combination of the biomarkers listed above with a combination of at least eight of the biomarkers selected from the group of CD3, CD4, CD8/CD8a, CD45, CD27, CD26, CD7, CD45RA and/or TIGIT for the determination if a cell is a Sézary signature cell.

When deciding if a cell is a Sézary signature cell, it is assumed that the sensitivity and specificity of the decision increases with the number of biomarkers that are used in the method of the invention. At the same time, the number of biomarkers that can be used in the method of the invention may be limited by the experimental method to determine the expression levels of the biomarkers and the availability of suitable binding agents. Accordingly, the invention relates to the method according to the invention, wherein 9 or more, biomarkers are used. That is, more than 8 biomarkers of the biomarkers of the group of biomarkers comprising CD28, CD197, CD3, HLA-ABC, CD194, CD26, CD164, CD44, CD45, CD45RA, CD45RO, CD7, CD4, CD27, TIGIT, PD-1, HLA-DR, CD14, CD158K, CD95, CD8/CD8a and CD195 may be used for deciding if a cell is a Sézary signature cell. Particularly, more than 8 biomarkers comprising the biomarker TIGIT and biomarkers of the group of biomarkers comprising CD28, CD197, CD3, HLA-ABC, CD194, CD26, CD164, CD44, CD45RA, CD7, CD4, CD27, PD-1, HLA-DR, CD14, CD158K, CD95, CD8/CD8a and CD195 may be used for deciding if a cell is a Sézary signature cell.

In certain embodiments, the method of the invention may be used for determining the relative frequency of mycosis fungoides cells in a plurality of cells.

Within the present invention, a "mycosis fungoides cell" is a cell that is indicative of mycosis fungoides. That is, if a certain relative frequency of mycosis fungoides cells is determined in a plurality of cells that has been obtained from a subject, the subject is diagnosed as having mycosis fungoides. Further, determining the relative frequency of mycosis fungoides cells in samples from the same subject at two or more time points allows to monitor the progression of mycosis fungoides in said subject.

The decision if a cell is a mycosis fungoides cell is based on the expression level of at least two, preferably four, more preferably six, biomarkers in said cell. In certain embodiments, the set of biomarkers that is used for the decision if a cell is a mycosis fungoides cell comprises 8, 9, 10, 11, 12, 13, 14, 15, 16, 17, 18, 19, 20, 21, 22, 23, 24, 25, 26, 27, 28, 29, 30, 31, 32, 33, 34, 35 or 36 biomarkers of the biomarkers listed in the claims. In other embodiments, the set of biomarkers that are used for the decision if a cell is a mycosis fungoides cell comprises further biomarkers that are not listed in Table 1.

That is, at least 9, at least 10, at least 11, at least 12, at least 13, at least 14, at least 15, at least 16, at least 17, at least 18, at least 19 or all 20 of the biomarkers CD28, CD197, CD3, HLA-ABC, CD194, CD26, CD164, CD44, CD45, CD45RA, CD45RO, CD7, CD4, CD27, TIGIT, PD-1, HLA-DR, CD14, CD158K, CD95, CD8/CD8a and CD195 may be used for the decision if a cell is a mycosis fungoides cell. Preferably, TIGIT is one of the biomarkers used for the decision if a cell is a mycosis fungoides cell.

In some embodiments, the disclosure encompasses any possible combination of the biomarkers listed above in particular any possible combination of the biomarkers listed above with a combination of at least nine of the biomarkers selected from the group of CD3, CD4, CD7, CD8/CD8a, CD45, CD27, CD26, CD45RA and TIGIT for the determination if a cell is a mycosis fungoides cell.

When deciding if a cell is a mycosis fungoides cell, it is assumed that the sensitivity and specificity of the decision increases with the number of biomarkers that are used in the method of the invention. At the same time, the number of biomarkers that can be used in the method of the invention is frequently limited by the experimental method to determine the expression levels of the biomarkers and the availability of suitable binding agents. Accordingly, the invention relates to the method according to the invention, wherein 9 or more biomarkers are used. That is, more than 8 biomarkers of the group of biomarkers comprising CD28, CD197, CD3, HLA-ABC, CD194, CD26, CD164, CD44, CD45, CD45RA, CD45RO, CD7, CD4, CD27, TIGIT, PD-1, HLA-DR, CD14, CD158K, CD95, CD8/CD8a and CD195 may be used for deciding if a cell is a mycosis fungoides cell. Particularly, more than 8 biomarkers comprising the biomarker TIGIT and biomarkers of the group of biomarkers comprising CD28, CD197, CD3, HLA-ABC, CD194, CD26, CD164, CD44, CD45RA, CD7, CD4, CD27, PD-1, HLA-DR, CD14, CD158K, CD95, CD8/CD8a and CD195 may be used for deciding if a cell is a mycosis fungoides cell.

In another embodiment, the invention relates to the method according to the invention, wherein a classifier algorithm is used to distinguish between a Sézary signature cell and a non-Sézary signature cell or a mycosis fungoides cell and a non-mycosis fungoides cell, respectively. The decision if a cell is a Sézary signature cell or a mycosis fungoides cell is based on the expression levels of a set of biomarkers. For that, the expression level of each biomarker from the set of biomarkers may be experimentally determined on a single cell level. The expression levels of the respective biomarkers may then be compared between cells when deciding if a cell is a Sézary signature cell or a mycosis fungoides cell, respectively. When deciding if a cell is a Sézary signature cell or a mycosis fungoides cell, one or more expression level thresholds may be defined for each biomarker in the set of biomarkers. For example, the expression level of a specific biomarker may indicate that a cell is a Sézary signature cell or a mycosis fungoides cell, respectively, if the expression level is below or above a defined threshold. This threshold may be defined based on the expression levels of said biomarker that have been previously determined in cells from healthy subjects, subjects that suffer from Sézary syndrome or mycosis fungoides, respectively, and/or subjects that suffer from other medical conditions. Accordingly, for each biomarker in a set of biomarkers, the expression level may be determined in a candidate cell and the expression level of this biomarker may then be compared to a defined threshold. A decision if a cell is a Sézary signature cell or a mycosis fungoides cell may be made by comparing the expression levels of all biomarkers from the set of biomarkers in a candidate cell to their respective thresholds. Thus, if the expression levels of a certain number of biomarkers in a set of biomarkers or of all biomarkers in a set of biomarkers indicate that a candidate cell is a Sézary signature cell or a mycosis fungoides cell, a decision may be made that the candidate cell is a Sézary signature cell or a mycosis fungoides cell, respectively.

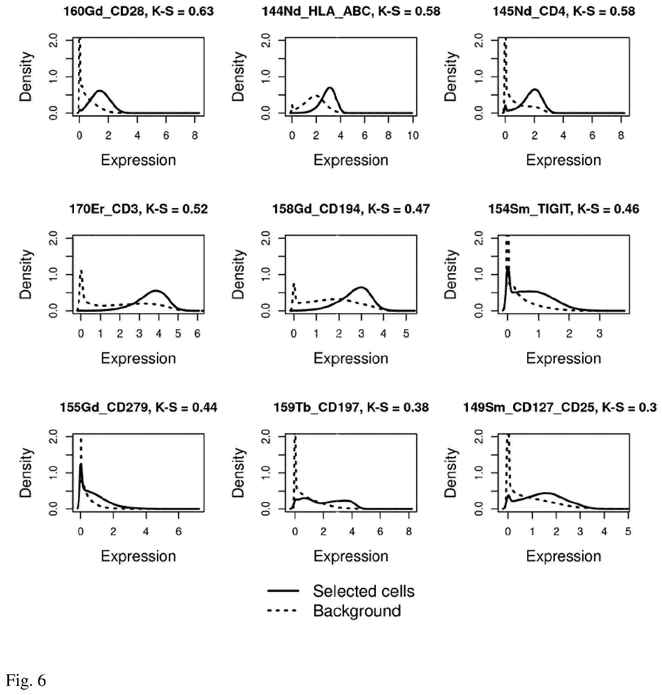

It is demonstrated in the Examples, that some biomarkers of the biomarkers listed in Table 1 are more relevant for diagnosing a certain medical condition, such as Sézary syndrome (see FIG. 6). Accordingly, a weight (or "filter weight") may be placed on the biomarkers in the set of biomarkers that reflects the relevance of each biomarker for a certain medical condition. That is, a biomarker that is more relevant for diagnosing a certain medical condition may be weighted more heavily than a biomarker that is less relevant for diagnosing the same medical condition. A biomarker with a higher weight or filter weight may then contribute more to the decision if a cell is a Sézary signature cell or a mycosis fungoides cell than a biomarker with a lower weight. If a biomarker is relevant for diagnosing a certain medical condition may be determined by analyzing the expression of said biomarker in subjects that suffer from said medical condition and/or subjects that do not suffer from said medical condition.

Comparing the expression level of biomarkers between cells and determining the relevance of each biomarker may be performed manually. However, it is preferred in the present invention that these steps are performed with the help of a classifier algorithm.

Within the present invention, a classifier algorithm may be used for distinguishing a Sézary signature cell from a non-Sézary signature cell or a mycosis fungoides cell from a non-mycosis fungoides cell, respectively. To be able to distinguish between these cell types, the classifier algorithm may be pre-trained with a training data set. That is, in certain embodiments, the classifier algorithm may be pre-trained with data sets that have been obtained with samples from known subjects. For example, when distinguishing Sézary signature cells from non-Sézary signature cells, the classifier algorithm may be pre-trained with data sets that have been generated with samples that have been obtained from subjects that are known to have Sézary syndrome and with data sets that have been generated with samples that have been obtained from subjects that are known not to have Sézary syndrome. Alternatively, when distinguishing mycosis fungoides cells from non-mycosis fungoides cells, the classifier algorithm may be pre-trained with data sets that have been generated with samples that have been obtained from subjects that are known to have mycosis fungoides and with data sets that have been generated with samples that have been obtained from subjects that are known not to have mycosis fungoides.

The training data set that is used for pre-training the classifier algorithm may comprise any number of data sets that have been generated with samples from individual subjects. Preferably, the training data set comprises at least 5, at least 6, at least 7, at least 8, at least 9, at least 10, at least 11, at least 12, at least 13, at least 14 or at least 15 data sets that have been generated with samples from individual subjects for each condition that is to be distinguished by the classifier algorithm, such as samples from Sézary syndrome patients and non-Sézary syndrome patients or mycosis fungoides patients and non-mycosis fungoides patients, respectively.

In another embodiment, the invention relates to the method according to the invention, wherein a convolutional neural network is used to distinguish between a Sézary signature cell and a non-Sézary signature cell or a mycosis fungoides cell and a non-mycosis fungoides cell, respectively.

Within the present invention, a convolutional neural network may be used for distinguishing a Sézary signature cell from a non-Sézary signature cell or a mycosis fungoides cell from a non-mycosis fungoides cell, respectively. However, it is preferred within the present invention, that the convolutional neural network CellCnn is used for distinguishing a Sézary signature cell from a non-Sézary signature cell or a mycosis fungoides cell from a non-mycosis fungoides cell, respectively. The CellCnn convolutional neural network has been described previously (Bodenmiller et al., Nat Biotechnol, 2012, 30(9), 858-867; Amir et al., Nat Biotechnol, 2013, 31(5), 545-552; Levine et al., Cell, 2015, 162(1), 184-197; Horowitz et al., Sci Transl Med, 2013, 5(208), 208ra145) and is publicly available (https://github.com/eiriniar/CellCnn). Further, it is described in the Examples how the CellCnn convolutional neural network may be used for distinguishing a Sézary signature cell from a non-Sézary signature cell or a mycosis fungoides cell from a non-mycosis fungoides cell, respectively.

In another embodiment, the invention relates to the method according to the invention, wherein the plurality of cells are human cells.

That is, the method of the invention is preferably used for the diagnosis of Sézary syndrome or mycosis fungoides in humans. Accordingly, it is preferred that the cells comprised in the plurality of cells are human cells. More preferably, the cells that are comprised in the plurality of cells are cells that have been obtained from the blood of a subject. Even more preferably, the cells that are comprised in the plurality of cells are peripheral blood mononuclear cells (PBMCs). The skilled person is aware of methods to obtain PBMCs from a subject. The subject may be any human subject, for example a healthy subject or a subject suffering from Sézary syndrome, mycosis fungoides or any other medical condition.

The term "PBMC" as used herein refers to peripheral blood mononuclear cells isolated from human peripheral blood preparations e.g. by use of a density gradient (e.g. Ficoll, PanColl). "PBMC" consists of lymphocytes and monocytes.

In another embodiment, the invention relates to the method according to the invention, wherein the levels of the biomarkers are determined using an antibody-based assay.

As mentioned above, it is preferred that the expression levels of the biomarkers are determined in an antibody-based assay. That is, any assay that comprises the use of antibodies and is suitable for determining the expression level of a biomarker may be used in the present invention. Preferably, antibodies are used that bind directly to the biomarker.

The term "antibody" as used herein includes whole antibodies and any antigen binding fragments (i.e., "antigen-binding portions") or single chains thereof. It also includes all other types of antibody-like molecules such as diabodies, triabodies, nanobodies and the like. An "antibody" refers to a glycoprotein comprising at least two heavy (H) chains and two light (L) chains inter-connected by disulfide bonds, or an antigen binding portion thereof. Each heavy chain is comprised of a heavy chain variable region (abbreviated herein as VH) and a heavy chain constant region. In certain naturally occurring antibodies, the heavy chain constant region is comprised of three domains, CHI, CH2 and CH3. In certain naturally occurring antibodies, each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen.

The term antibody as used herein comprises IgG1, IgG2, IgG3, IgG4, IgM, IgA1, IgA2, IgD, and IgE antibodies. Antibodies typically bind specifically to their cognate antigen with high affinity, reflected by a dissociation constant (KD) of 10⁻⁵ to 10⁻¹¹ M or less. Any KD greater than about 10⁻⁴ M is generally considered to indicate nonspecific binding. The phrase "antigen-binding portion" of an antibody, as used herein, refers to one or more fragments of an antibody that retain the ability to specifically bind to an antigen (e.g., a biomarker of the present invention). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the VL, V H, CL and CHI domains; (ii) a F(ab')2 fragment, a bivalent fragment comprising two Fab fragments linked by a disulfide bridge at the hinge region; (iii) a Fd fragment consisting of the VH and CHI domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR) or (vii) a combination of two or more isolated CDRs which may optionally be joined by a synthetic linker. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. These antibody fragments are obtained using conventional techniques known to those with skill in the art, and the fragments are screened for utility in the same manner as are intact antibodies. Antigen-binding portions can be produced by recombinant DNA techniques, or by enzymatic or chemical cleavage of intact immunoglobulins.

Within the present invention, the antibodies are preferably labeled to facilitate detection and/or quantification of a biomarker. For example, antibodies may be labeled with a fluorophore to allow detection and/or quantification of biomarkers in flow cytometry-based assays or metal isotopes to allow detection and/or quantification of biomarkers in mass cytometry-based assays. Accordingly, in another embodiment, the invention relates to the method according to the invention, wherein the antibody-based assay is an antibody-based flow cytometry or mass cytometry assay.

Within the present invention, it is preferred that the expression levels of all biomarkers in Table 1 are used when deciding if a cell is a Sézary signature cell or a non-Sézary signature cell or when deciding if a cell is a mycosis fungoides cell or a non-mycosis fungoides cell, respectively. However, due to restrictions in the experimental setup, fewer biomarkers may be used for the diagnosis of Sézary syndrome or mycosis fungoides with high specificity and sensitivity. When only a certain set of biomarkers from the biomarkers in Table 1 is used for the diagnosis of Sézary syndrome or mycosis fungoides, it is preferred that these biomarkers from Table 1 that are highly indicative, e.g. that have a high filter weight, for a respective medical condition are considered.

The examples show that it is possible to diagnose Sézary syndrome in patients with high sensitivity by comparing the expression levels of a reduced set of 6 to 10 highly indicative biomarkers (see Table 3).

Accordingly, in another embodiment, the invention relates to the method according to the invention, wherein biomarkers are r) a panel comprising or consisting of nine different biomarkers, namely (i) CD3, (ii) CD4, (iii) CD7, (iv) CD8/CD8a, (v) CD26, (vi) CD27, (vii) CD45, (viii) CD45RA and (ix) TIGIT; u) a panel comprising or consisting of ten different biomarkers, namely (i) CD3, (ii) CD4, (iii) CD7, (iv) CD8/CD8a, (v) CD26, (vi) CD45RO, (vii) TIGIT, (viii) CD194, (ix) CD197 and (x) PD-1.

The examples further show that a panel of biomarkers comprising the biomarkers CD3, CD4, CD7, CD8/CD8a, CD45, CD27, CD26, CD45RA and TIGIT, or a selection thereof, is particularly advantageous for use according to the invention (Figure 7).

Certain biomarkers described in Table 7, such as, CD3, CD4, CD8/CD8a, and/or CD45 are particularly useful for the initial separation of cells and certain biomarkers described in Table 7, such as, CD27, CD26, CD7, CD45RA and/or TIGIT are particularly useful for the specialized separation of cells (Figure 7) according to the invention.

In certain embodiments, some or all of the biomarkers that are particularly useful for the specialized separation of cells are present on a smaller fraction of cells in a plurality of cells according to the invention (e.g., a sample) compared to some or all of the biomarkers that are particularly useful for the initial separation of cells.

In some embodiments the initial cell separation process uses method that is biomarker independent (e.g., exploiting physical cell properties (e.g. filtration, sedimentation, centrifugation methods such as density gradient centrifugation), cell behavior (e.g. adhesion), (micro)fluidic cell separation, depletion cells, micromanipulation, limiting dilution). In some embodiments, the initial cell separation process comprises or consists of a positive selection for immune cells. In some embodiments, the initial cell separation process comprises or consists of a positive selection for T-cells. In some embodiments, the initial cell separation process comprises or consists of a negative selection for cytotoxic T cells.

In some embodiments the initial cell separation process comprises determination of at least one other biomarker, preferably at least one other biomarker selected from Table 1.

In some embodiments, the initial cell separation process comprises or consists of a negative selection for CD8⁺ cells.

In a preferred embodiment, a panel of nine biomarkers may consist of biomarkers CD3, CD4, CD7, CD8/CD8a, CD26, CD27, CD45, CD45RA and TIGIT.

In a preferred embodiment, a panel of ten biomarkers may consist of biomarkers CD3, CD4, CD7, CD8/CD8a, CD26, CD45RO, TIGIT, CD194, CD197 and PD-1.

In other embodiments, the panel of biomarkers may comprise any panel of biomarkers described above and in addition one or more further biomarkers listed in Table 1. In other embodiments, the panel of biomarkers may comprise all 36 biomarkers listed in Table 1.

Panels may be adapted to obtain adapted panels for reliably diagnosing Sézary syndrome and/or mycosis fungoides in a subject with high sensitivity and specificity, wherein the adapted panel consists of the same or a lower number of biomarkers by a method comprising the steps of:
(i) adding one or more biomarkers to a panel of the current invention to obtain an alternative panel;
(ii) placing weight (e.g. as learned by CellCnn) to the biomarkers of the alternative panel by testing the alternative panel on a set of samples with known classification for a a. Sézary signature cell or a non-Sézary signature cell; and/or b. a mycosis fungoides cell and a non-mycosis fungoides cell;
(iii) excluding one or more biomarkers of an absolute weight below the average weight of the biomarkers of the alternative panel to obtain a provisional adapted panel;
(iv) verifying the specificity and selectivity using a validation data set to identify adapted panels.

In step (i) of the method to obtain an adapted panel, the biomarker(s) to be added to the panel can be any biomarker but is/are preferably (a) biomarker(s) selected from the group listed in Table 1. In some embodiments of the invention, (one of) the biomarker(s) to be added to the panel of the current invention is known to be characteristic for a same cell type as one of the biomarkers of the panel of the current invention. In some embodiments of the invention, the biomarker(s) to be added to the panel may be chosen based on various reasons, including but not limited to economic reasons, availability of reagents and compatibility with the measurement equipment.

In step (ii) of the method to obtain an adapted panel, placing a weight may be done using CellCnn as described in the examples, or using any suitable weighting method known to the skilled person. The full alternative panel and/or a certain number of the biomarkers of the alternative panel can be tested to obtain information for placing a weight to the biomarkers. For example alternative panel-minus-one controls may be used to obtain information regarding weighting (e.g., as described by Tung, James W et al. Clinics in laboratory medicine vol. 27,3 (2007): 453-68).

In step (iii) of the method to obtain an adapted panel, the biomarker with the lowest weight may be excluded.

In step (iv) of the method to obtain an adapted panel, the specificity and selectivity of the provisional adapted panel may be verified as described in the examples. Panels that have a specificity and selectivity for the differential diagnosis of Sézary syndrome and/or mycosis fungoides as opposed to atopic dermatitis and/or other medical conditions, below a certain specificity and selectivity threshold, are excluded.

In another embodiment, the invention relates to a computer-implemented method for determining the frequency of Sézary signature cells or mycosis fungoides cells, the method comprising the steps of: (i) executing a classifier algorithm on a set of data comprising the levels of expression of eight or more, more preferably nine or more, biomarkers, wherein the biomarkers comprise or consist of
a) CD28, CD44, HLA_ABC, CD26, CD3, CD195, CD164, CD8/CD8a, HLA-DR, CD194, CD11b, CD38, CD45, CD95, CD223, CD45RA, CD14, CD7, CD123, TIM3, TIGIT, CD19, and FcRL3, b) CD28, PD-1, CD4, CD3, HLA ABC, TIGIT, CD197, CD27, CD26, CD44, CD194, CD7, CD45RA, CD158k, and CD164, c) CD3, CD4, CD7, CD8/CD8a, CD26, CD27, CD45, CD45RA and TIGIT, or d) CD3, CD4, CD7, CD8/CD8a, CD26, CD45RO, TIGIT, CD194/CCR4, CD197/CCR7, and CD279/PD-1, in a plurality of cells; (ii) determining the frequency of Sézary signature cells or mycosis fungoides cells, respectively, in the plurality of cells underlying the set of data based on the levels of expression of the biomarkers.

That is, the invention further relates to a computer-implemented method that can be used for determining the frequency of Sézary signature cells or mycosis fungoides cells, respectively. The computer-implemented method comprises a classifier algorithm that can be used for distinguishing between a Sézary syndrome signature cell and a non-Sézary signature cell or a mycosis fungoides cell and a non-mycosis fungoides cell, respectively. Preferably, the classifier algorithm is a classifier algorithm that has been pre-trained with a data set that has been generated with samples that have been obtained from known subjects. That is, it is preferred that the classifier algorithm is a classifier algorithm that has been pre-trained with data sets from healthy subjects and with data sets from subjects that suffer from Sézary syndrome, mycosis fungoides or any other medical condition.

The biomarkers that are analyzed in the computer-implemented method are preferably any panel of biomarkers that is described above that falls under the definition of the invention.

In another embodiment, the invention relates to the method according to the invention, wherein the classifier algorithm comprises one or a combination of a support vector algorithm, a convolutional neural network, a tree-based method, logistic regression.

That is the classifier algorithm may be any classifier algorithm known in the art that is suitable for determining the relative frequency of Sézary signature cells or mycosis fungoides cell. However, it is preferred that the classifier algorithm comprises a support vector algorithm, a convolutional neural network, a tree-based method or a logistic regression, or any combination thereof.

In another embodiment, the disclosure relates to a computer program product containing instructions for performing the computer-implemented method according to the invention. In another embodiment, the invention relates to a method for diagnosing a subject as having Sézary syndrome or as having mycosis fungoides, the method comprising the steps of: (i) determining the frequency of Sézary signature cells or mycosis fungoides cells, respectively, in a sample obtained from the subject using the method according to the invention; (ii) comparing the frequency of Sézary signature cells or mycosis fungoides cells, respectively, determined in step (i) to the frequency of Sézary signature cells or mycosis fungoides cells, respectively, in a sample that has been obtained from a subject not suffering from Sézary syndrome or mycosis fungoides, respectively, and/or to the frequency of Sézary signature cells or mycosis fungoides cells, respectively, in a sample that has been obtained from a subject with Sézary syndrome or mycosis fungoides, respectively; and (iii) determining a subject as having Sézary syndrome or mycosis fungoides, respectively, if the frequency of Sézary signature cells or mycosis fungoides cells, respectively, determined in step (ii) is higher compared to the frequency of Sézary signature cells or mycosis fungoides cells, respectively, for the subject not suffering from Sézary syndrome or mycosis fungoides, respectively, and/or if the frequency of Sézary signature cells or mycosis fungoides cells, respectively, determined in step (ii) is similar or higher compared to the frequency of Sézary signature cells or mycosis fungoides cells, respectively, in the sample that has been obtained from the subject with Sézary syndrome or mycosis fungoides, respectively.

Determining the frequency of Sézary signature cells or mycosis fungoides cells in a plurality of cells that have been obtained from a subject may allow diagnosing said subject with Sézary syndrome or mycosis fungoides, respectively. For that, in a first step, the frequency of Sézary signature cells or mycosis fungoides cells in a sample that has been obtained from a subject may be determined with the method of the present invention. The obtained frequency may then in a second step be compared to the frequency of Sézary signature cells or mycosis fungoides cells from known subjects.

For example, the frequency of Sézary signature cells in a sample that has been obtained from an unknown subject may be compared to the frequency of Sézary signature cells in a sample that has been obtained from a subject that is not suffering from Sézary syndrome. In this case, the unknown subject may be diagnosed with Sézary syndrome, if the frequency of Sézary signature cells is higher in the sample that has been obtained from the unknown subject compared to the frequency of Sézary signature cells in the sample that has been obtained from the subject that is not suffering from Sézary syndrome.

Alternatively, the frequency of Sézary signature cells in a sample that has been obtained from an unknown subject may be compared to the frequency of Sézary signature cells in a sample that has been obtained from a subject that is suffering from Sézary syndrome. In this case, the unknown subject may be diagnosed with Sézary syndrome, if the frequency of Sézary signature cells is similar or higher in the sample that has been obtained from the unknown subject compared to the frequency of Sézary signature cells in the sample that has been obtained from the subject that is suffering from Sézary syndrome.

In certain embodiments, the frequency of Sézary signature cells in a sample that has been obtained from an unknown subject may be compared to the frequencies of Sézary signature cells in samples that have been obtained from subjects suffering from Sézary syndrome and from subjects not suffering from Sézary syndrome.

The frequency of Sézary signature cells used to determine whether a subject suffers from Sézary syndrome may also be obtained from known frequencies. That is, the determined frequency may be compared to a known value, e.g. a pre-determined threshold value.

In other embodiments, the method of the invention may be used for the diagnosis of mycosis fungoides. In this case, the frequency of mycosis fungoides cells in a sample that has been obtained from an unknown subject may be compared to the frequency of mycosis fungoides cells in a sample that has been obtained from a subject that is not suffering from mycosis fungoides. In this case, the unknown subject may be diagnosed with mycosis fungoides, if the frequency of mycosis fungoides cells is higher in the sample that has been obtained from the unknown subject compared to the frequency of mycosis fungoides cells in the sample that has been obtained from the subject that is not suffering from mycosis fungoides.

Alternatively, the frequency of mycosis fungoides cells in a sample that has been obtained from an unknown subject may be compared to the frequency of mycosis fungoides cells in a sample that has been obtained from a subject that is suffering from mycosis fungoides. In this case, the unknown subject may be diagnosed with mycosis fungoides, if the frequency of mycosis fungoides cells is similar or higher in the sample that has been obtained from the unknown subject compared to the frequency of mycosis fungoides cells in the sample that has been obtained from the subject that is suffering from mycosis fungoides.

In certain embodiments, the frequency of mycosis fungoides cells in a sample that has been obtained from an unknown subject may be compared to the frequencies of mycosis fungoides cells in samples that have been obtained from subjects suffering from mycosis fungoides and from subjects not suffering from mycosis fungoides.

The frequency of mycosis fungoides cells used to determine whether a subject suffers from mycosis fungoides may also be obtained from known frequencies. That is, the determined frequency may be compared to a known value, e.g. a pre-determined threshold value.

In another embodiment, the invention relates to the method according to the invention, wherein determining the subject as having Sézary syndrome or mycosis fungoides, respectively, comprises the use of a classifier algorithm.

That is, the comparison of the frequencies of Sézary signature cells or mycosis fungoides cells between samples that have been obtained from different subjects may be performed manually, or may comprise the use of a classifier algorithm. That is, the comparison may comprise the use of a separate classifier algorithm that is specifically used for the comparison of frequencies of cells or the classifier algorithm may be comprised in the classifier algorithm that is used for distinguishing between Sézary signature cells and non-Sézary signature cells or between mycosis fungoides cells and non-mycosis fungoides cells, respectively.

In another embodiment, the invention relates to the method according to the invention, wherein the classifier algorithm comprises a convolutional neural network and/or logistic regression.

The classifier algorithm that is used for comparing the relative frequency of specific cell types preferably comprises a convolutional neural network and/or logistic regression.

In another embodiment, the invention relates to the method according to the invention, wherein the subject without Sézary syndrome is a healthy subject or a subject suffering from atopic dermatitis, non-specific dermatitis, erythroderma and/or mycosis fungoides.

In certain embodiments, the frequency of Sézary signature cells may be compared between samples to distinguish subjects having Sézary syndrome from healthy subjects. However, the method of the present invention may also be used to distinguish subjects suffering from Sézary syndrome from subjects suffering from other medical conditions, in particular medical conditions that result in similar symptoms as Sézary syndrome. For example, the method of the present invention may be used to distinguish between subjects that suffer from Sézary syndrome and subjects that suffer from atopic dermatitis, non-specific dermatitis, erythroderma and/or mycosis fungoides.

The terms "atopic dermatitis" or "non-specific dermatitis" as used herein relate to an inflammatory skin condition characterized by chronic pruritus, lichenification, xerosis, erythematous papules and plaques.

Erythroderma is an inflammatory skin disease with redness and scaling that affects nearly the entire cutaneous surface. Erythroderma is generalized exfoliative dermatitis, which involves 90% or more of the patient's skin. The most common cause of erythroderma is exacerbation of an underlying skin disease, such as psoriasis, contact dermatitis, seborrheic dermatitis, lichen planus, pityriasis rubra pilaris or a drug reaction, such as the use of topical steroids.

In another embodiment, the invention relates to the method according to the invention further comprising one or more other diagnostic tests and/or additional information about the subject.

That is, the method of the invention for diagnosing Sézary syndrome or mycosis fungoides may be combined with other methods known in the art to improve the diagnostic results.

In another embodiment, the invention relates to the method according to the invention, wherein the one or more other diagnostic tests is/are selected from the group consisting of: whole-body imaging tests, skin lesion biopsies, histopathology tests, CD4/CD8 ratio determination, cell count analysis and PCR analysis of T cell receptor clonality.

In certain embodiments, the method of the invention may be combined with whole-body imaging tests. Whole-imaging tests comprise for example, examination of the skin by the eye or with suitable devices such as scanners, cameras, or magnifying glasses. Further whole-body imaging tests comprise the use of CT scan, PET scan and MRI.

In other embodiments, the method of the invention may be combined with skin lesion biopsies. A skin lesion biopsy comprises the removal of a skin lesion and the subsequent examination of that skin lesion, with the goal to diagnose a certain medical condition. The skin lesion may be examined by any method known in the art. Preferably, the skin lesion is analyzed by microscopic techniques. The term "skin lesion biopsies" comprises any type of biopsy, such as shave biopsy, punch biopsy, excisional biopsy, incisional biopsy, or Mohs microsurgery.

Another classical test used in the diagnosis of Sézary syndrome or mycosis fungoides is determination of the CD4/CD8 ratio in a subject. The skilled person is aware of methods to determine the CD4/CD8 ratio in a subject. According to previous reports, an elevated CD4/CD8 ratio is indicative of Sézary syndrome and/or mycosis fungoides.

In other embodiments, the method according to the invention may be combined with cell count analysis. In case of Sézary syndrome, a cell count of 1000 Sézary cells per mm³ peripheral blood is considered to be indicative of Sézary syndrome. The skilled person is aware of methods to obtain peripheral blood from a subject and to quantify the numbers of specific cell types.

In addition, the method according to the invention may be combined with PCR analysis of T cell receptor clonality as described previously (Wood et al., J Invest Dermatol., 1994, 103(1), 34-41).

The method according to the invention may be combined with any of the above-mentioned methods in any combination.

In another embodiment, the invention relates to the method according to the invention, wherein the additional information about the subject comprises age and/or clinical information.

Further, the diagnosis of a subject as having Sézary syndrome or mycosis fungoides may further involve information about the age and/or other clinical information of said subject. That is, Sézary syndrome is diagnosed most commonly in adults over age 60. Mycosis fungoides is diagnosed most commonly in adults over age 50.

In another embodiment, the invention relates to a method for determining the susceptibility to treatment of Sézary syndrome or mycosis fungoides in a subject, the method comprising the steps of: (i) determining the frequency of Sézary signature cells or mycosis fungoides cells, respectively, in a first sample that has been obtained from said subject; (ii) determining the frequency of Sézary signature cells or mycosis fungoides cells, respectively, in a second sample that has been obtained from the same subject, wherein the second sample has been obtained at a later time point than the first sample; (iii) determining the change in frequency of Sézary signature cells or mycosis fungoides cells, respectively, between the first and second sample; and (iv) determining the subject as being susceptible to treatment of Sézary syndrome or mycosis fungoides, respectively, if the frequency of Sézary signature cells or mycosis fungoides cells, respectively, is lower in the second sample than in the first sample; wherein the frequency of Sézary signature cells or mycosis fungoides cells, respectively, is determined using the method according to the invention.

Besides using the method of the invention for the diagnosis of Sézary syndrome or mycosis fungoides, the method of the invention may also be used for monitoring the progression of the respective disease or for determining the susceptibility to a treatment of the respective disease.

For that, the frequency of Sézary signature cells or mycosis fungoides cells may be determined in at least two samples that have been obtained from the same subject at different time points. By comparing the relative frequencies of Sézary signature cells or mycosis fungoides cells between the at least two samples, the progression of Sézary syndrome or mycosis fungoides, respectively, in that subject or the susceptibility of a treatment against Sézary syndrome or mycosis fungoides, respectively, in said subject may be analyzed.

That is, a subject is said to be susceptible to a treatment of Sézary syndrome or mycosis fungoides, if the frequency of Sézary signature cells or mycosis fungoides cells, respectively, is lower at a later time point, e.g. after a treatment or intervention, compared to an earlier time point, e.g. before said treatment or intervention. Accordingly, Sézary syndrome or mycosis fungoides is said to be progressing or worsening in a subject, if the frequency of Sézary signature cells or mycosis fungoides cells, respectively, is higher at a later time point compared to an earlier time point.

The comparison of frequencies of Sézary signature cells or mycosis fungoides cells between samples may be performed as described above. That is, the comparison may be made manually, or may involve a classifier algorithm as described above.

In another embodiment, the invention relates to the method according to the invention, wherein the sample is a blood sample, in particular, wherein the sample comprises peripheral blood mononuclear cells.

That is, the plurality of cells is preferably obtained from a human blood sample. More preferably, the sample comprises or consists of peripheral blood mononuclear cells. The skilled person is aware of methods to obtain peripheral blood mononuclear cells from a subject.

The present disclosure also encompasses biomarker panels or compositions comprising reagents for the detection of biomarkers, that can be used for the diagnosis of Sézary syndrome and/or mycosis fungoides.

In one embodiment the invention relates to a composition consisting of reagents for the detection of biomarkers, for the diagnosis of Sézary syndrome and/or mycosis fungoides, the panel consisting of: a) CD3, CD4, CD7, CD8/CD8a, CD26, CD27, CD45, CD45RA and TIGIT; or b) CD3, CD4, CD7, CD8/CD8a, CD26, CD45RO, TIGIT, CD194, CD197 and PD-1.

In one embodiment the invention relates to a composition consisting of reagents for the detection of biomarkers, for the diagnosis of Sézary syndrome and/or mycosis fungoides, the panel consisting of: CD3, CD4, CD7, CD8/CD8a, CD26, CD27, CD45, CD45RA and TIGIT.

In one embodiment the invention relates to a composition consisting of reagents for the detection of biomarkers, for the diagnosis of Sézary syndrome and/or mycosis fungoides, the panel consisting of: CD3, CD4, CD7, CD8/CD8a, CD26, CD45RO, TIGIT, CD194, CD197 and PD-1.

The examples show that it is possible to diagnose Sézary syndrome in a subject with high sensitivity by comparing the expression levels of a reduced set of 6 to 10 highly indicative biomarkers (see Table 3).

In other embodiments, a composition consisting of reagents for the detection of nine biomarkers, may be used in the diagnosis of Sézary syndrome. In other embodiments, a panel of nine biomarkers may consist of the biomarkers CD3, CD4, CD7, CD8/CD8a, CD26, CD27, CD45, CD45RA and TIGIT or a composition may consist of reagents for the detection of nine biomarkers namely CD3, CD4, CD7, CD8/CD8a, CD26, CD27, CD45, CD45RA and TIGIT.

In other embodiments, a panel of ten biomarkers may consist of the biomarkers CD3, CD4, CD7, CD8/CD8a, CD26, CD45RO, TIGIT, CD194, CD197 and PD-1 or a composition may consist of reagents for the detection of ten biomarkers namely CD3, CD4, CD7, CD8/CD8a, CD26, CD45RO, TIGIT, CD194, CD197 and PD-1.

The present specification also discloses kits, in particular research kits. In order to carry out the method of the invention, the kit can be prepared by collecting necessary reagents.

The kit may comprise compositions comprising reagents for the detection of biomarkers, a reaction container and specifications. By the use of such a kit, it becomes possible to carry out the sample preparation and or the method for determining the frequency of Sézary signature cells and/or mycosis fungoides cells in a plurality of cells by merely adding a sample to the aforementioned reaction container. The kit can be used by adding the sample and/or the ingredients of the composition comprising reagents for the detection of biomarkers to the reaction container in a certain sequence.

The kits (to be prepared in context) of this disclosure or the methods and uses of the invention may further comprise or be provided with (an) instruction manual(s). For example, said instruction manual(s) may guide the skilled person (how) to employ the kit in the diagnostic uses provided herein and in accordance with the present invention. Particularly, said instruction manual(s) may comprise guidance to use or apply the herein provided methods or uses.

In other embodiments, the panel of biomarkers may comprise in addition one or more further biomarkers listed in Table 1. In other embodiments, the panel of biomarkers may comprise all 36 biomarkers listed in Table 1.

In a further embodiment, the disclosure relates to a method of treating a subject suffering from Sézary syndrome or mycosis fungoides, the method comprising the use of one or more binding molecules selectively binding to a biomarker comprising or consisting of
a) CD28, CD44, HLA-ABC, CD26, CD3, CD195, CD164, CD8/CD8a, HLA-DR, CD194, CD11b, CD38, CD45, CD95, CD223, CD45RA, CD14, CD7, CD123, TIM3, TIGIT, CD19, and FcRL3,
b) CD28, PD-1, CD4, CD3, HLA-ABC, TIGIT, CD197, CD27, CD26, CD44, CD194, CD7, CD45RA, CD158k, and CD164,
c) CD3, CD4, CD7, CD8/CD8a, CD26, CD27, CD45, CD45RA and TIGIT, or
d) CD3, CD4, CD7, CD8/CD8a, CD26, CD45RO, TIGIT, CD194/CCR4, CD197/CCR7, and CD279/PD-1.

### Brief description of Figures

FIG. 1: Accuracies of predictions from CellCnn models trained on all 36 markers on held-out data for 10-fold cross-validation. Median accuracy is 1.0.
FIG. 2: Confusion matrix for training data (left) and validation data (right), calculated based on predictions of a CellCnn model trained using all 36 markers and all samples from the discovery cohort. 11 samples were set aside as validation data during the training process.
FIG. 3: Boxplots comparing the relative frequency of selected cells using a filter response cutoff of 0.3 between non-SS and SS samples. (p = 1.6e-13, Wilcoxon rank-sum test).
FIG. 4: t-SNE maps of cells from all samples in the discovery cohort (5000 cells randomly subsampled from each sample), showing in black either cells selected as above the filter response cutoff (left) or cells manually annotated as CD4 T cells (right).
FIG. 5: Learned filter weights for each marker from the trained CellCnn model. Positive scores correspond to markers contribute positively towards a predictive of Sézary syndrome, while negative scores correspond to markers that contribute negatively towards a prediction of Sézary syndrome.
FIG. 6: Smoothed densities of normalized, arcsinh-transformed marker expression for the top 9 most informative markers in selected cells (solid line) vs. all measured cells (dotted line). K-S indicates the Kolmogorov-Smirnov test statistic for each comparison. Markers are shown in decreasing order of separation between the selected cells distribution and the background distribution.
Fig. 7: Display of workflow according to analysis strategy for Panel A as disclosed herein.

### Examples

Aspects of the present invention are additionally described by way of the following illustrative non-limiting examples that provide a better understanding of embodiments of the present invention and of its many advantages. The following examples are included to demonstrate preferred embodiments of the invention. It should be appreciated by those of skill in the art that the techniques disclosed in the examples which follow represent techniques used in the present invention to function well in the practice of the invention, and thus can be considered to constitute preferred modes for its practice.

### Identification of a Sézary-specific biomarker from discovery data

A discovery cohort of 60 PBMC samples were collected from 60, comprising 20 samples from patients with clinically-diagnosed Sézary Syndrome (SS), 20 samples from patients with clinically-diagnosed Atopic Dermatitis (AD), and 20 healthy donor (HD) samples. CyTOF data was acquired from these samples using a panel of 37 antibodies (Table 1a), which were selected for the identification of the major human immune cell subsets in blood as well as known or suspected cancer-specific markers.

PBMCs were isolated from whole blood via standard Ficoll isolation and 3x10⁶ cells per sample were resuspended in Maxpar Cell Staining buffer (CSB, Fluidigm). To stain for viable cells, samples were incubated for 10 min at room temperature (RT) with Cisplatin in PBS (1:20'000 dilution, Cell-ID Cisplatin-194Pt/198Pt, Fluidigm). For fixation and permeabilisation, the samples were resuspended in Maxpar Fix I Buffer (Fluidigm) and incubated for 10 minutes (min) at RT, followed by two washing steps with the Barcode Perm Buffer (Fluidigm). For barcoding, the samples were mixed with the barcodes (Cell-IP 20-Plex Pd Barcoding kit, Fluidigm), resuspended with 100ul of Barcode Perm buffer in advance. After 30 min of incubation at RT, samples were centrifuged and washed with Maxpar Staining Buffer. Finally, all barcoded samples were pooled together. The pooled barcoded sample was stained with the antibody panel for 20 min at RT and washed twice with CSB. For the intercalator staining, 200 µl of freshly prepared 4% PFA in PBS was added to the stained cells and samples were incubated for 24h at 4°C, followed by iridium staining by adding 200 µl of freshly prepared intercalator solution (1:5000 dilution in Max Par Fix and Perm buffer, Cell-ID Intercalator-Ir, Fluidigm) for 1 hour at RT. Before CyTOF acquisition, the samples were mixed with 15% of EQ^{™} Four Element Calibration Beads (¹⁴⁰Ce, ¹⁵¹Eu, ¹⁵³Eu, ¹⁶⁵Ho, ¹⁷⁵Lu, Fluidigm).

The data was pre-processed by applying bead normalization to the entire dataset (Chevrier, S. et al._Cell Syst. 6, 612-620.e5 (2018) and Finck, R. et al. Cytometry A 83A, 483-494 (2013)) and then applying an arcsinh transformation with a cofactor of 5 (Nowicka, M. et al. F1000Research 6, 748 (2019)). Samples were de-barcoded using the CATALYST package in R (Chevrier, S. et al._Cell Syst. 6, 612-620.e5 (2018) and Zunder, E. R. et al. Nat. Protoc. 10, 316-333 (2015)). After de-barcoding, the inventors observed that 7 samples (5 SS, 1 AD and 1 HD) contained less than 10,000 events. The inventors reasoned that these samples, which had been previously observed to be of low quality during data acquisition, might not include all relevant rare cell types, or, in the worst case, might introduce biases into the results; therefore, the inventors excluded them from further analysis.

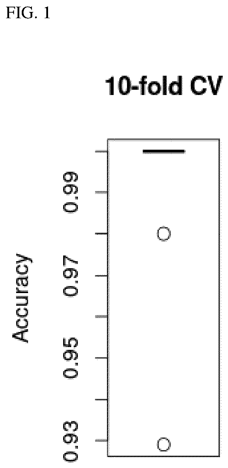

The remaining 53 samples were used to train a CellCnn neural network to distinguish between SS and non-SS samples. For this task, AD and HD samples were combined into a "non-SS" class. To first evaluate the robustness of the CellCnn model on this dataset, the inventors conducted 10-fold cross-validation. The median accuracy on held-out test data across 10 runs for each two-way comparison of SS vs. non-SS samples was 1.0 (Figure 1). From these results, the inventors concluded that CellCnn was able to robustly identify a Sézary-specific biomarker from the discovery cohort. The inventors then proceeded to train a CellCnn model using the full discovery dataset.

Input data for CellCnn consisted of 1000 batches of 3000 cells each chosen randomly from each sample independently. For CyTOF data, all measured protein markers were included as parameters to train the network. For FACS data, all measured protein markers plus forward-and side-scatter parameters were included to train the network. Each sample was given a label of either 0, corresponding to Atopic Dermatitis and Healthy donor samples, or 1, corresponding to Sézary syndrome samples. CellCnn was run in classification mode. During training, 20% of the samples were set aside for validation, chosen in a stratified manner to maintain the relative proportions of each class. Fifteen independent networks were generating using different hyperparameters randomly chosen from the following options:
∘ Number of filters: [3, 4, 5, 6, 7, 8, 9, 10]
∘ MaxPool percentage: [0.01, 1, 5, 20, 100]
∘ Learning rate: range(0.001, 0.01)

All other hyperparameters were fixed at the default value. Each network was trained for a maximum of 50 epochs, or until the validation loss no longer increased for 5 consecutive epochs.

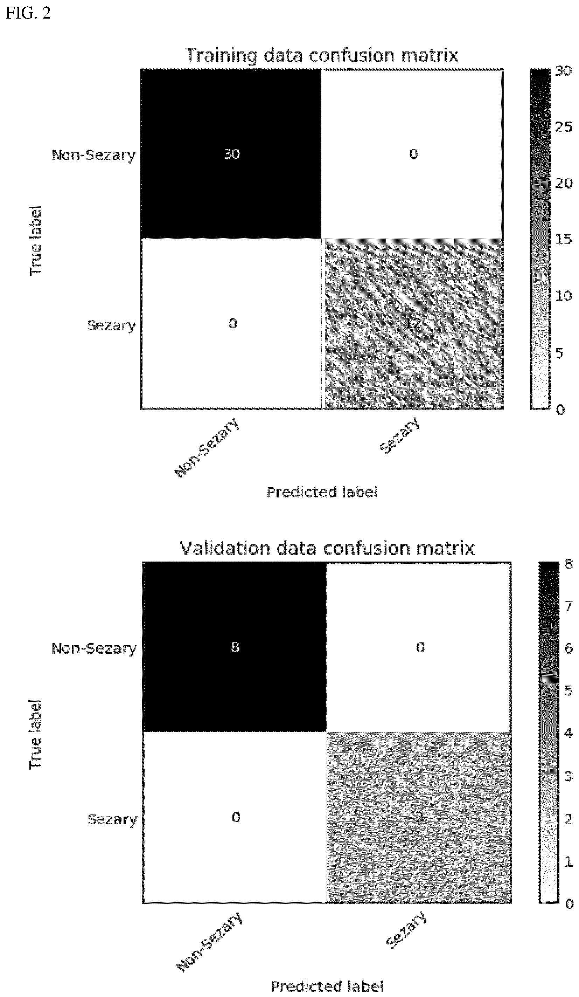

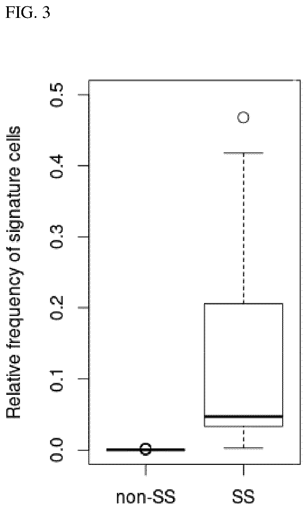

The resulting model showed 100% accuracy on both training and validation data (Figure 2). Using the learned weights from the CellCnn filter most strongly positively associated with SS samples, the inventors calculated a filter response score for every cell in the dataset. These scores can be used to set a threshold to determine the Sézary-specific signature cells. A range of thresholds were tested, and the threshold that best separated SS and non-SS samples by relative frequency of Sézary signature cells was chosen as 0.3 (Figure 3). The median frequency of signature cells in SS samples was 12.9% of all live PBMCs, while the median frequency in non-SS samples was 0.033% (p = 1.6e-13, Wilcoxon rank-sum test).

### Validation of the discovered signature in an independent patient cohort

In order to validate the Sézary-specific biomarker, PBMCs were collected from an independent cohort of 33 individuals, comprising 11 patients with Sézary syndrome, 11 patients with Atopic Dermatitis, and 11 healthy donors. Sample labels were blinded during the data acquisition and analysis phases. The validation samples were pre-processed in the same way as the discovery samples, with bead normalization performed on all samples together to reduce the potential batch effects between the two cohorts. The pre-trained CellCnn network was used to predict the status (SS or non-SS) of each sample, which was then compared against the corresponding clinically-diagnosed disease status. The predictions showed a sensitivity of 0.81 and a specificity of 0.95.

Performance metrics for the sample classification task were calculated as follows. True positives (TP) were defined as SS samples that were predicted as SS samples. False positives (FP) were defined as AD or HD samples that were predicted as SS samples. True negatives (TN) were defined as AD or HD samples that were predicted as AD or HD samples. False negatives (FN) were defined as SS samples that were predicted as AD or HD. Sensitivity was calculated as TP/(TP+FN); specificity as TN/(FP+TN); accuracy as (TP+TN)/(TP+FP+TN+FN); positive predictive value (PPV) as TP/(TP+FP); and negative predictive value (NPV) as TN/(TN+FN). The F1-score was calculated as the harmonic mean of sensitivity and specificity, using the formula 2*(sensitivity * specificity) / (sensitivity + specificity).

### Phenotypic characterization of Sézary signature cells

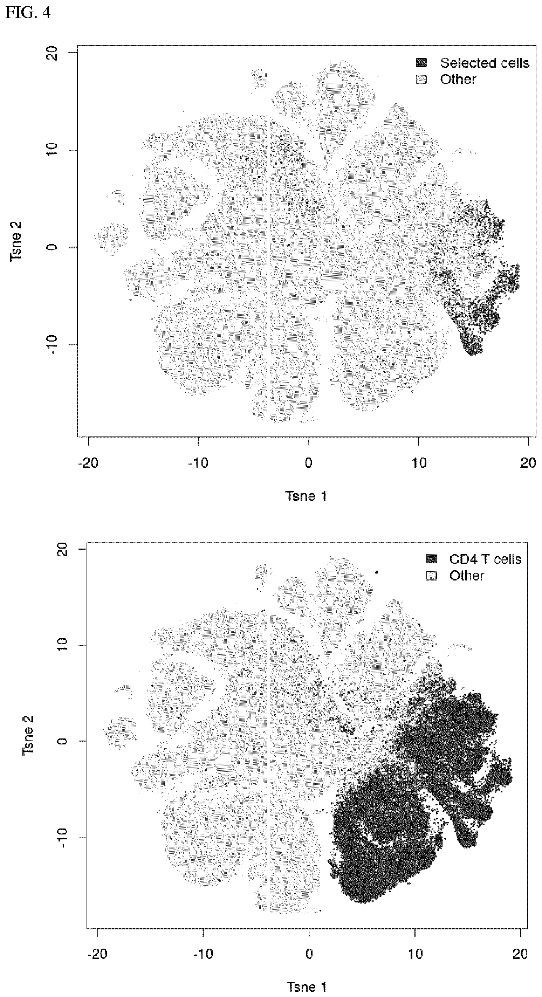

In parallel to the CellCnn analysis, the inventors performed clustering on the CyTOF data using FlowSOM (Van Gassen, S. et al., Cytometry A 87, 636-645 (2015)), followed by manual annotation of the resulting 100 nodes into major immune cell lineages based on marker expression profiles (CD4 T cells, CD8/CD8a T cells, other T cells, B cells, NK cells, myeloid cells and pDCs). T cells, and more specifically CD4+ T cells, showed an enrichment in SS vs. non-SS samples, while all other identified cell types showed a relative depletion. These results are consistent with the expected immune profiles from Sézary syndrome patients, as it is a disease originating from a CD4+ T cell expansion. Plotting the CellCnn-derived Sézary signature cells on a tSNE map revealed that they largely overlap with the CD4+ T cell cluster (Figure 4); however, not all CD4+ T cells were included in the Sézary signature cells. In order to better phenotypically characterize the Sézary signature cells, the inventors examined both the learned filter weights from CellCnn and the distribution of marker expression in those cells.

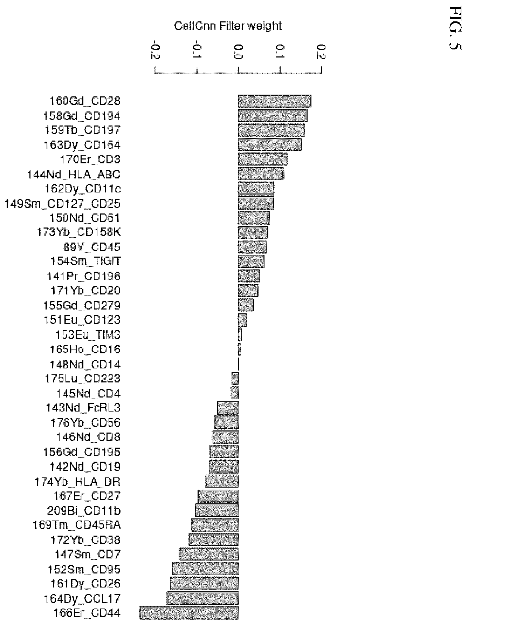

The filters learned by CellCnn correspond to a set of weights for each measured protein marker, which can be either positive or negative (Figure 5). Taken as a whole, these weights may be considered to define the molecular profile of a theoretical group of cells that is strongly predictive of a given class label. Both positive and negative weights may be informative, as positive weights describe markers that have high values (are relatively enriched) in these idealized cells, while negative weights describe markers that have low values (are relatively depleted) in the same cells as compared to the entire dataset. As the Sézary signature cells were selected based on their filter response scores, which indicate how similar their individual expression profiles are to the learned filter, it is expected that signature cells should show enrichment for markers with high filter weights and depletion for markers with low filter weights. Nonetheless, the expression levels of markers in individual cells are heterogeneous, making it appropriate to examine their overall distributions.

For each marker examined, the inventors compared the expression distribution in Sézary signature cells vs. the expression distribution in the entire dataset using a Kolmogorov-Smirnov 2-sample test (Figure 6). The inventors performed one set of tests with the null hypothesis that the expression distribution in signature cells is stochastically greater than the background distribution, in order to identify markers most enriched in signature cells, and another set of tests with the null hypothesis that the distribution in signature cells is stochastic smaller than the background distribution, in order to identify markers most depleted in signature cells. The inventors then compared the lists of markers ranked by the Kolmogorov-Smirnov test statistic with the list of markers ranked by filter weights (Table 2). Among the top 10 enriched markers in signature cells, 7 (CD28, HLA-ABC, CD3, CD4, CD194, CD197, and CD127/CD25) were also in the top 10 markers with the most positive filter weights. Among the top 10 depleted markers in signature cells, 7 (CD44, CD38, CD7, CD11b, CD45RA, HLA-DR, and CD26) were also in the top 10 markers with the most negative filter weights.

### Definition of reduced antibody panels

Based on these results, as well as on expert knowledge of expected lineage markers present in T cells and other immune cell types, the inventors designed several panels comprising a reduced number of antibodies. These panels were tested *in silico* by retraining CellCnn using only the data corresponding to the markers in each reduced panel. As with the initial full antibody panel, the inventors conducted 10-fold cross-validation and calculated the accuracies of predictions on the held-out test data for each run. Median accuracies for the different reduced panels ranged from 0.96 to 1.00 (Table 3). The inventors also trained a single CellCnn model using all of the samples from the discovery cohort for each reduced panel, and used these models to predict the labels of the validation cohort samples. The predictions showed sensitivities ranging from 0.64 to 1.00 and specificities ranging from 0.32 to 1.00.

### Preferred panels

Preferred panels of biomarkers to be measured consist of a combination of both biomarkers whose levels are increased in cells specific to patients with Sézary syndrome ("positive biomarkers") and biomarkers whose levels are decreased in cells specific to patients with Sézary syndrome ("negative biomarkers"). Panels of particular diagnostic utility comprise the panels listed in Table 3. The preferred panels may be expanded by adding one or more additional biomarkers from Table 1. This may result in increased sensitivity and/or specificity for diagnosis.

### Comparative sensitivity of marker panels

Several example panels of antibodies were tested. For each, thresholds for every marker individually were identified and then the frequencies of the resulting selected cells in SS and non-SS samples were calculated, before the p-value comparing those frequencies using a Wilcoxon test was determined. The same was done using CellCnn by defining a threshold on a CellCnn filter score. As can be seen in Table 4, all panels were able to separate the classes of samples.

In addition, marker panels 1 to 19 were used for a comparison of the sensitivity of the various panels vs. four existing methods from the prior art. There were 15 patients in this cohort. As can be seen in Table 5, the tested panels were able to determine samples as comprising SS cells which were not detected using one of the methods of the prior art.

While aspects of the invention are illustrated and described in detail in the Figures and in the foregoing tables and description, such Figures, tables and description are to be considered illustrative or exemplary and not restrictive. Also reference signs in the claims should not be construed as limiting the scope.

Whenever the word "comprising" is used in the claims, it should not be construed to exclude other elements or steps. It should also be understood that the terms "essentially", "substantially", "about", "approximately" and the like used in connection with an attribute or a value may define the attribute or the value in an exact manner in the context of the present disclosure. The terms "essentially", "substantially", "about", "approximately" and the like could thus also be omitted when referring to the respective attribute or value. The terms "essentially", "substantially", "about", "approximately" when used with a value may mean the value ±10%, preferably ±5%.

**Table 1a**

| **Target** | **Clone** | **Mass** | **Element** |
|---|---|---|---|
| CD45 | HI30 | 89 | Y |
| CD196/CCR6 | G034E3 | 141 | Pr |
| CD19 | HIB19 | 142 | Nd |
| CD307c/FcRL3 | H5/FcRL3 | 143 | Nd |
| HLA-ABC | W6/32 | 144 | Nd |
| CD4 | RPA-T4 | 145 | Nd |
| CD8 / CD8a | RPA-T8 | 146 | Nd |
| CD7 | CD7-6B7 | 147 | Sm |
| CD14 | RMO52 | 148 | Nd |
| CD25 (IL-2R) | 2A3 | 149 | Sm |
| CD61 | VI-PL2 | 150 | Nd |
| CD123 | 6H6 | 151 | Eu |
| CD95 [Fas] | DX2 | 152 | Sm |
| CD366 [Tim3] | F38-2E2 | 153 | Eu |
| TIGIT | MBSA43 | 154 | Sm |
| CD279/PD-1 | EH12.2H7 | 155 | Gd |
| CD195/CCR5 | NP-6G4 | 156 | Gd |
| CD194/CCR4 | 205410 | 158 | Gd |
| CD197/CCR7 | G043H7 | 159 | Tb |
| CD28 | CD28.2 | 160 | Gd |
| CD26 | BA5b | 161 | Dy |
| CD11c | Bu15 | 162 | Dy |
| CD164 | 67D2 | 163 | Dy |
| CCL17/CADM1 | 3E1 | 164 | Dy |
| CD16 | 3G8 | 165 | Ho |
| CD44 | BJ18 | 166 | Er |
| CD27 | O323 | 167 | Er |
| CD127/IL-7Ra | A019D5 | 168 | Er |
| CD45RA | HI100 | 169 | Tm |
| CD3 | UCHT1 | 170 | Er |
| CD20 | 2H7 | 171 | Yb |
| CD38 | HIT2 | 172 | Yb |
| KIR3DL2/CD158k | 539304 | 173 | Yb |
| HLA-DR | L243 | 174 | Yb |
| CD223 [LAG3] | 11C3C65 | 175 | Lu |
| CD56 | NCAM16.2 | 176 | Yb |

**Table 2. Comparison of marker rankings**

| **Marker** | **Rank by filter weight** | **Rank by K-S test statistic (enrichment)** | **Rank by K-S test statistic (depletion)** |
|---|---|---|---|
| CD28 | 1 | 1 | 34 |
| CD194 | 2 | 5 | 31 |
| CD197 | 3 | 8 | 36 |
| CD164 | 4 | 10 | 23 |
| CD3 | 5 | 4 | 25 |
| HLA-ABC | 6 | 2 | 32 |
| CD11c | 7 | 19 | 10 |
| CD127_CD25 | 8 | 9 | 24 |
| CD61 | 9 | 33 | 2 |
| CD158K | 10 | 12 | 29 |
| CD45 | 11 | 14 | 18 |
| TIGIT | 12 | 6 | 28 |
| CD196 | 13 | 23 | 11 |
| CD20 | 14 | 17 | 17 |
| PD-1 | 15 | 7 | 35 |
| CD123 | 16 | 24 | 19 |
| TIM3 | 17 | 22 | 22 |
| CD16 | 18 | 20 | 21 |
| CD14 | 19 | 27 | 4 |
| CD223 | 20 | 15 | 26 |
| CD4 | 21 | 3 | 33 |
| FcRL3 | 22 | 16 | 27 |
| CD56 | 23 | 21 | 16 |
| CD8/CD8a | 24 | 26 | 12 |
| CD195 | 25 | 36 | 14 |
| CD19 | 26 | 25 | 13 |
| HLA-DR | 27 | 28 | 8 |
| CD27 | 28 | 11 | 30 |
| CD11b | 29 | 29 | 6 |
| CD45RA | 30 | 35 | 7 |
| CD38 | 31 | 30 | 3 |
| CD7 | 32 | 32 | 5 |
| CD95 | 33 | 18 | 20 |
| CD26 | 34 | 34 | 9 |
| CCL17 | 35 | 13 | 15 |
| CD44 | 36 | 31 | 1 |

**Table 3. Preferred biomarker panels**

| **Panel** | **Median accuracy (10-fold CV, discovery cohort)** | **S.D. accuracy (10-fold CV, discovery cohort)** | **Sensitivity (validation cohort)** | **Specificity (validation cohort)** |
|---|---|---|---|---|
| CD28, CD197, CD3, HLA-ABC, CD194, CD26 | 0.83 | 0.12 | 0.91 | 0.86 |
| CD28, CD197, CD194, CD3, HLA-ABC, CD164, CD44, CD45RA, CD26, CD7 | 1.00 | 0.12 | 0.91 | 0.91 |
| CD28, CD197, CD4, CD3, HLA-ABC, CD27, TIGIT, PD-1, HLA-DR, CD14 | 0.79 | 0.30 | 1.00 | 0.32 |
| CD28, CD197, CD4, CD3, HLA-ABC, CD27, TIGIT, PD-1 | 0.82 | 0.18 | 0.64 | 1.00 |
| CD28, CD197, CD4, CD3, HLA-ABC, CD27, TIGIT, PD-1, CD26 | 1.00 | 0.14 | 0.82 | 0.73 |
| CD28, CD197, CD4, CD3, HLA- | 0.92 | 0.19 | 0.73 | 1.00 |
| ABC, CD27, TIGIT, PD-1, CD164 | | | | |
| CD28, CD197, CD4, CD3, HLA-ABC, CD27, TIGIT, PD-1, CD158K | 0.75 | 0.23 | 0.64 | 1.00 |
| CD28, CD197, CD4, CD3, HLA-ABC, CD27, TIGIT, PD-1, CD164, CD158K | 0.79 | 0.23 | 0.80 | 1.00 |
| CD28, CD197, CD3, HLA-ABC, CD194, CD26, CD7 | 1.00 | 0.12 | 0.82 | 0.91 |
| CD28, CD197, CD3, HLA-ABC, CD194, CD26, CD44 | 0.92 | 0.10 | 1.00 | 0.86 |
| CD28, CD3, HLA-ABC, CD194, CD26, CD44, CD95, CD8/CD8a, CD164, CD195 | 1.00 | 0.19 | 1.00 | 0.96 |

**Table 4 - Threshold values for biomarker panels**

| **Pa nel** | **Marke r** | **Co mp.** | **Threshold** | **Mean freq. non-SS** | **Mean freq. SS** | **P-value Wilcox** | **CellCnn threshold** | **Mean freq. non-SS** | **Mean freq. SS** | **P-value Wilcox** |
|---|---|---|---|---|---|---|---|---|---|---|
| | | | | | | | | | | |
| 1 | CD28 | >= | 1,15 | 1,12 | 10,61 | 0,00381 | 0,0286 | 0,108 | 2,47 | 1,49E-04 |
| | HLA-ABC | >= | 2,81 | | | | | | | |
| | | | | | | | | | | |
| 2 | HLA-ABC | >= | 3 | 0,975 | 11,57 | 0,0031 | 0,00514 | 0,00716 | 0,0145 | 0,0118 |
| | CD3 | >= | 2,03 | | | | | | | |
| | | | | | | | | | | |
| 3 | CD28 | >= | 0,971 | 1,05 | 11,56 | 0,0016 | 0,217 | 0,027 | 0,286 | 8,59E-09 |
| | HLA-ABC | >= | 2,76 | | | | | | | |
| | CD3 | >= | 2,01 | | | | | | | |
| | | | | | | | | | | |
| 4 | CD28 | >= | 0,995 | 0,526 | 11,21 | 0,000149 | 0,0836 | 0,303 | 7,27 | 1,05E-05 |
| | HLA-ABC | >= | 2,66 | | | | | | | |
| | CD3 | >= | 2,03 | | | | | | | |
| | CD26 | <= | 1,72 | | | | | | | |
| | | | | | | | | | | |
| 5 | CD28 | >= | 0,971 | 0,74 | 10,94 | 0,00332 | 0,345 | 0,345 | 7,1 | 8,56E-04 |
| | HLA-ABC | >= | 2,76 | | | | | | | |
| | CD4 | >= | 0,575 | | | | | | | |
| | HLA-DR | <= | 1,13 | | | | | | | |
| | | | | | | | | | | |
| 6 | CD28 | >= | 0,971 | 0,346 | 11,51 | 5,87E-08 | 0,0762 | 0,844 | 16,44 | 4,45E-11 |
| | CD44 | <= | 1,6 | | | | | | | |
| | HLA-ABC | >= | 2,42 | | | | | | | |
| | CD3 | >= | 1,73 | | | | | | | |
| | CD26 | <= | 1,91 | | | | | | | |
| | | | | | | | | | | |
| 7 | CD28 | >= | 0,995 | 0,499 | 10,79 | 0,000149 | 0,248 | 0,829 | 13,04 | 3,10E-07 |
| | HLA-ABC | >= | 2,66 | | | | | | | |
| | CD3 | >= | 1,72 | | | | | | | |
| | CD26 | <= | 2,03 | | | | | | | |
| | CD194 | >= | 0,674 | | | | | | | |
| | | | | | | | | | | |
| 8 | CD28 | >= | 0,971 | 0,258 | 11,12 | 3,62E-09 | 0,115 | 0,0724 | 6,24 | 6,63E-10 |
| | CD44 | <= | 1,6 | | | | | | | |
| | HLA-ABC | >= | 2,42 | | | | | | | |
| | CD3 | >= | 1,73 | | | | | | | |
| | CD26 | <= | 1,91 | | | | | | | |
| | CD195 | <= | 1,41 | | | | | | | |
| | | | | | | | | | | |
| 9 | CD28 | >= | 0,8 | 0,811 | 11,45 | 0,002879 | 0,0171 | 0,0958 | 2,63 | 0,0001349 |
| | HLA-ABC | >= | 2,76 | | | | | | | |
| | CD3 | >= | 1,75 | | | | | | | |
| | CD4 | >= | 0,575 | | | | | | | |
| | CD164 | >= | 3,86 | | | | | | | |
| | PD-1 | <= | 4,41 | | | | | | | |
| | | | | | | | | | | |
| 10 | CD28 | >= | 0,995 | 0,479 | 10,81 | 0,0001226 | 0,0986 | 0,348 | 11,6 | 6,63E-10 |
| | HLA-ABC | >= | 2,66 | | | | | | | |
| | CD26 | <= | 1,72 | | | | | | | |
| | CD3 | >= | 2,03 | | | | | | | |
| | CD 194 | >= | 0,674 | | | | | | | |
| | CD7 | <= | 3,71 | | | | | | | |
| | | | | | | | | | | |
| 11 | CD28 | >= | 1,15 | 0,885 | 10,19 | 0,00216 | 0,0567 | 0,0555 | 1,08 | 5,87E-08 |
| | HLA-ABC | >= | 2,82 | | | | | | | |
| | CCL17 | <= | 3,27 | | | | | | | |
| | CD127 | <= | 3,51 | | | | | | | |
| | CD16 | <= | 1,31 | | | | | | | |
| | CD20 | <= | 1,26 | | | | | | | |
| | | | | | | | | | | |
| 12 | HLA-ABC | >= | 2,91 | 0,752 | 11,71 | 0,000856 | 0,102 | 0,0346 | 0,312 | 5,09E-10 |
| | CD3 | >= | 2,03 | | | | | | | |
| | CD11b | <= | 0,644 | | | | | | | |
| | CD38 | <= | 0,971 | | | | | | | |
| | CD56 | <= | 1,61 | | | | | | | |
| | CD223 | <= | 2,01 | | | | | | | |
| | | | | | | | | | | |
| 13 | CD28 | >= | 1,15 | 0,862 | 9,847 | 0,00609 | 0,415 | 0,03 | 0,568 | 4,52E-09 |
| | HLA-ABC | >= | 2,78 | | | | | | | |
| | CD45 | >= | 0,704 | | | | | | | |
| | CD45 | <= | 4,4 | | | | | | | |
| | CD196 | <= | 2,64 | | | | | | | |
| | CD19 | <= | 1,23 | | | | | | | |
| | FcRL3 | <= | 2,09 | | | | | | | |
| | CD25 | <= | 3,88 | | | | | | | |
| | | | | | | | | | | |
| 14 | CD28 | >= | 1,26 | 1,93 | 9,69 | 0,00288 | 0,194 | 3,26 | 13,8 | 1,33E-05 |
| | TIGIT | <= | 3,08 | | | | | | | |
| | CD61 | <= | 2,07 | | | | | | | |
| | CD3 | >= | 1,31 | | | | | | | |
| | TIM3 | <= | 1,32 | | | | | | | |
| | CD11c | <= | 3,28 | | | | | | | |
| | CD123 | <= | 1,45 | | | | | | | |
| | | | | | | | | | | |
| | | | | | | | | | | |
| 15 | CD28 | >= | 0,971 | 0,672 | 10,47 | 0,002495 | 0,075 | 0,039 | 0,928 | 2,65E-06 |
| | HLA-ABC | >= | 2,76 | | | | | | | |
| | HLA-DR | <= | 1,2 | | | | | | | |
| | CD4 | >= | 0,0959 | | | | | | | |
| | CD14 | <= | 1,72 | | | | | | | |
| | CD3 | >= | 1,21 | | | | | | | |
| | PD-1 | <= | 4,41 | | | | | | | |
| | | | | | | | | | | |
| 16 | CD28 | >= | 0,971 | 0,301 | 11,09 | 1,58E-08 | 0,681 | 0,394 | 13,7 | 3,87E-10 |
| | CD44 | <= | 1,59 | | | | | | | |
| | HLA-ABC | >= | 2,42 | | | | | | | |
| | CD3 | >= | 1,39 | | | | | | | |
| | CD26 | <= | 1,91 | | | | | | | |
| | CD194 | >= | 0,674 | | | | | | | |
| | CD197 | >= | 0,0569 | | | | | | | |
| | | | | | | | | | | |
| 17 | CD28 | >= | 0,943 | 0,367 | 12,1 | 1,11E-09 | 0,338 | 0,226 | 13,8 | 6,24E-11 |
| | CD44 | <= | 1,59 | | | | | | | |
| | HLA-ABC | >= | 2,21 | | | | | | | |
| | CD3 | >= | 1,39 | | | | | | | |
| | CD26 | <= | 1,91 | | | | | | | |
| | CD164 | >= | 3,86 | | | | | | | |
| | CD194 | >= | 0,674 | | | | | | | |
| | CD45R A | <= | 4,39 | | | | | | | |
| | CD7 | <= | 3,71 | | | | | | | |
| | | | | | | | | | | |
| 18 | CD28 | >= | 0,73 | 0,185 | 11,53 | 4,45E-11 | 0,548 | 0,592 | 20,5 | 8,60E-10 |
| | CD44 | <= | 1,62 | | | | | | | |
| | HLA-ABC | >= | 2,34 | | | | | | | |
| | CD3 | >= | 1,39 | | | | | | | |
| | CD26 | <= | 1,91 | | | | | | | |
| | CD195 | <= | 1,44 | | | | | | | |
| | CD164 | >= | 4,39 | | | | | | | |
| | CD8/C D8a | <= | 1,62 | | | | | | | |
| | CD194 | >= | 0,546 | | | | | | | |
| | CD95 | <= | 1,71 | | | | | | | |
| | | | | | | | | | | |
| 19 | CD28 | >= | 0,73 | 0,146 | 11,58 | 1,81E-09 | 0,205 | 0,273 | 17,8 | 6,40E-13 |
| | CD44 | <= | 1,62 | | | | | | | |
| | HLA_ ABC | >= | 2,09 | | | | | | | |
| | CD26 | <= | 1,91 | | | | | | | |
| | CD3 | >= | 1,39 | | | | | | | |
| | CD195 | <= | 1,44 | | | | | | | |
| | CD164 | >= | 4,39 | | | | | | | |
| | CD8/C D8a | <= | 1,62 | | | | | | | |
| | HLA-DR | <= | 1,22 | | | | | | | |
| | CD194 | >= | 0,546 | | | | | | | |
| | CD11b | <= | 0,716 | | | | | | | |
| | CD38 | <= | 2,63 | | | | | | | |
| | CD45 | >= | 0,478 | | | | | | | |
| | CD95 | <= | 1,71 | | | | | | | |
| | CD223 | <= | 2,01 | | | | | | | |
| | CD45R A | <= | 4,64 | | | | | | | |
| | CD14 | <= | 2,02 | | | | | | | |
| | CD7 | <= | 3,71 | | | | | | | |
| | CD123 | <= | 1,45 | | | | | | | |
| | TIM3 | <= | 1,45 | | | | | | | |
| | TIGIT | <= | 3,08 | | | | | | | |
| | CD19 | <= | 1,37 | | | | | | | |
| | FcRL3 | <= | 2,09 | | | | | | | |

**Table 5 - Detection frequency and sensitivity of panels of Table 4**

| **Method** | # **Sézary patients detected out of 15-patient cohort** | **Sensitivity** |
|---|---|---|
| **CD4/CD8 ratio** | 10 | 0,666666667 |
| **% CD4 CD7-** | 8 | 0,533333333 |
| **% CD4 CD26-** | 11 | 0,733333333 |
| **TCR clone (flow)** | 11 | 0,733333333 |
| **Panel 1** | 8 | 0,533333333 |
| **Panel 2** | 9 | 0,6 |
| **Panel 3** | 8 | 0,533333333 |
| **Panel 4** | 13 | 0,866666667 |
| **Panel 5** | 9 | 0,6 |
| **Panel 6** | 14 | 0,933333333 |
| **Panel 7** | 13 | 0,866666667 |
| **Panel 8** | 14 | 0,933333333 |
| **Panel 9** | 13 | 0,866666667 |
| **Panel 10** | 14 | 0,933333333 |
| **Panel 11** | 15 | 1 |
| **Panel 12** | 14 | 0,933333333 |
| **Panel 13** | 12 | 0,8 |
| **Panel 14** | 11 | 0,733333333 |
| **Panel 15** | 12 | 0,8 |
| **Panel 16** | 14 | 0,933333333 |
| **Panel 17** | 15 | 1 |
| **Panel 18** | 14 | 0,933333333 |
| **Panel 19** | 15 | 1 |

Using the algorithm as described herein, 15 markers were shortlisted (Table 6) based on their contribution to detecting Sézary cells in Sézary Syndrome (SS) patients. Expression of these markers in SS patients as well as control samples was measured using flow cytometry (FACS) technology. Subsequently, the most preferred markers of a reduced set of markers were identified based on the FACS results. The analysis resulted in the preferred panels of 9 and 10 markers, respectively, as shown in Tables 7 and 9, respectively.

**Table 6:**

| |
|---|
| CD28 |
| PD-1 |
| CD4 |
| CD3 |
| HLA ABC |
| TIGIT |
| CD197 |
| CD27 |
| CD26 |
| CD44 |
| CD194 |
| CD7 |
| CD45RA |
| CD158k |
| CD164 |

**Table 7 - Panel A:**

| |
|---|
| CD3 |
| CD4 |
| CD7 |
| CD8/CD8a |
| CD26 |
| CD27 |
| CD45 |
| CD45RA |
| TIGIT |

Three data analysis strategies for manual gating of the data were identified, wherein the strategies are used sequentially:
**Strategy 1:** CD45+/CD3+ or CD3low/CD4+ broad/CD26-TIGIT+ | Freq. of CD3+ or CD45+ Positive SS, if frequency of selected cells is >= XX%
   If negative, continue with Strategy 2
**Strategy 2:** CD45+/CD3+ or CD3low/CD4+ broad/CD7low | Freq. of CD3+ or CD45+ Positive SS, if frequency of selected cells is >= XX%
   If negative, continue with Strategy 3
**Strategy 3:** CD45+/CD3+ or CD3low/CD4+ broad/CD27+CD45RA+/TIGIT+ | Freq. of CD27+CD45RA+
   Positive SS, if frequency of selected cells is >= XX%
   If negative, patient is negative for SS

By applying these manual gating strategies, Panel A demonstrated accuracy as indicated in Table 8. For comparison, specificity and sensitivity of the CellCnn algorithm achieved on the same dataset is indicated, too.

**Table 8:**

| Strategy | Specificity, % | Sensitivity, % |
|---|---|---|
| Strategy 1 | 100 | 76 |
| Strategy 1 | 100 | 86 |
| + | | |
| Strategy 2 | | |
| Strategy 1 | 100 | 95 |
| + | | |
| Strategy 2 | | |
| + | | |
| Strategy 3 | | |
| CellCnn algorithm | 100 | 71 |

Panel A performance was determined by analysing FACS data of 21 samples of SS patients and 52 control samples.

**Table 9 - Panel B:**

| |
|---|
| CD3 |
| CD4 |
| CD7 |
| CD8/CD8a |
| CD26 |
| CD45RO |
| TIGIT |
| CD194/CCR4 |
| CD197/CCR7 |
| CD279/PD-1 |

## Claims

1. A method for determining the frequency of Sézary signature cells and/or mycosis fungoides cells in a plurality of cells, the method comprising the steps of:
i) determining the levels of expression of 9 or more biomarkers in a plurality of cells; and
ii) determining the frequency of Sézary signature cells and/or mycosis fungoides cells in the plurality of cells based on the levels of expression of the 9 or more, biomarkers;
wherein the biomarkers comprise or consist of
a) CD28, CD44, HLA-ABC, CD26, CD3, CD195, CD164, CD8/CD8a, HLA-DR, CD194, CD11b, CD38, CD45, CD95, CD223, CD45RA, CD14, CD7, CD123, TIM3, TIGIT, CD19, and FcRL3,
b) CD28, PD-1, CD4, CD3, HLA-ABC, TIGIT, CD197, CD27, CD26, CD44, CD194, CD7, CD45RA, CD158k, and CD164,
c) CD3, CD4, CD7, CD8/CD8a, CD26, CD27, CD45, CD45RA and TIGIT, or
d) CD3, CD4, CD7, CD8/CD8a, CD26, CD45RO, TIGIT, CD194/CCR4, CD197/CCR7, and CD279/PD-1.

2. The method according to any one of claim 1, wherein a classifier algorithm is used to distinguish between a Sézary signature cell and a non-Sézary signature cell or a mycosis fungoides cell and a non-mycosis fungoides cell, respectively.

3. The method according to any one of claims 1 to 2, wherein a convolutional neural network is used to distinguish between a Sézary signature cell and a non-Sézary signature cell or a mycosis fungoides cell and a non- mycosis fungoides cell, respectively.

4. The method according to any one of claims 1 to 3, wherein the plurality of cells are human cells.

5. The method according to any one of claims 1 to 4, wherein the levels of the biomarkers are determined using an antibody-based assay

6. The method according to claim 5, wherein the antibody-based assay is an antibody-based flow cytometry or mass cytometry assay.

7. The method according to any one of claims 1 to 6, wherein the 9 or more biomarkers are a panel comprising or consisting of nine different biomarkers, namely (i) CD3, (ii) CD4, (iii) CD7, (iv) CD8/CD8a, (v) CD26, (vi) CD27, (vii) CD45, (viii) CD45RA and (ix) TIGIT.

8. A computer-implemented method for determining the frequency of Sézary signature cells or mycosis fungoides cells, the method comprising the steps of:
i) executing a classifier algorithm on a set of data comprising the levels of expression of 9 or more biomarkers in a plurality of cells;
ii) determining the frequency of Sézary signature cells or mycosis fungoides cells, respectively, in the plurality of cells underlying the set of data based on the levels of expression of the 9 or more, biomarkers;
wherein the biomarkers comprise or consist of
a) CD28, CD44, HLA_ABC, CD26, CD3, CD195, CD164, CD8/CD8a, HLA-DR, CD194, CD11b, CD38, CD45, CD95, CD223, CD45RA, CD14, CD7, CD123, TIM3, TIGIT, CD19, and FcRL3,
b) CD28, PD-1, CD4, CD3, HLA ABC, TIGIT, CD197, CD27, CD26, CD44, CD194, CD7, CD45RA, CD158k, and CD164,
c) CD3, CD4, CD7, CD8/CD8a, CD26, CD27, CD45, CD45RA and TIGIT, or
d) CD3, CD4, CD7, CD8/CD8a, CD26, CD45RO, TIGIT, CD194/CCR4, CD197/CCR7, and CD279/PD-1;
preferably, wherein the classifier algorithm comprises one or a combination of a support vector algorithm, a convolutional neural network, a tree-based method, logistic regression.

9. A method for diagnosing a subject as having Sézary syndrome or as having mycosis fungoides, the method comprising the steps of:
i) determining the frequency of Sézary signature cells or mycosis fungoides cells, respectively, in a sample obtained from the subject using the method according to any one of claims 1 to 7;
ii) comparing the frequency of Sézary signature cells or mycosis fungoides cells, respectively, determined in step (i) to the frequency of Sézary signature cells or mycosis fungoides cells, respectively, in a sample that has been obtained from a subject not suffering from Sézary syndrome or mycosis fungoides, respectively, and/or to the frequency of Sézary signature cells or mycosis fungoides cells, respectively, in a sample that has been obtained from a subject with Sézary syndrome or mycosis fungoides, respectively; and
iii) determining a subject as having Sézary syndrome or mycosis fungoides, respectively, if the frequency of Sézary signature cells or mycosis fungoides cells, respectively, determined in step (ii) is higher compared to the frequency of Sézary signature cells or mycosis fungoides cells, respectively, for the subject not suffering from Sézary syndrome or mycosis fungoides, respectively, and/or if the frequency of Sézary signature cells or mycosis fungoides cells, respectively, determined in step (ii) is similar or higher compared to the frequency of Sézary signature cells or mycosis fungoides cells, respectively, in the sample that has been obtained from the subject with Sézary syndrome or mycosis fungoides, respectively.

10. The method according to claim 9,
a) wherein determining the subject as having Sézary syndrome or mycosis fungoides, respectively, comprises the use of a classifier algorithm;
b) wherein the classifier algorithm comprises a convolutional neural network and/or logistic regression;
c) wherein the subject without Sézary syndrome is a healthy subject or a subject suffering from atopic dermatitis, non-specific dermatitis, erythroderma and/or mycosis fungoides;
d) wherein the sample is a blood sample, in particular, wherein the sample comprises peripheral blood mononuclear cells; and/or
e) further comprising one or more other diagnostic tests and/or additional information about the subject, preferably wherein the one or more other diagnostic tests is/are selected from the group consisting of: whole-body imaging tests, skin lesion biopsies, histopathology tests, CD4/CD8 ratio determination, cell count analysis and PCR analysis of T cell receptor clonality; and/or
wherein the additional information about the subject comprises age and/or clinical information.

11. A method for determining the susceptibility to treatment of Sézary syndrome or mycosis fungoides in a subject, the method comprising the steps of:
i) determining the frequency of Sézary signature cells or mycosis fungoides cells, respectively, in a first sample that has been obtained from said subject;
ii) determining the frequency of Sézary signature cells or mycosis fungoides cells, respectively, in a second sample that has been obtained from the same subject, wherein the second sample has been obtained at a later time point than the first sample;
iii) determining the change in frequency of Sézary signature cells or mycosis fungoides cells, respectively, between the first and second sample; and
iv) determining the subject as being susceptible to treatment of Sézary syndrome or mycosis fungoides, respectively, if the frequency of Sézary signature cells or mycosis fungoides cells, respectively, is lower in the second sample than in the first sample;
wherein the frequency of Sézary signature cells or mycosis fungoides cells, respectively, is determined using the method according to any one of claims 1 to 7, preferably wherein the sample is a blood sample, in particular, wherein the sample comprises peripheral blood mononuclear cells.

12. A composition consisting of reagents for the detection of biomarkers for the diagnosis of Sézary syndrome or mycosis fungoides, the biomarkers consisting of
a) CD28, CD44, HLA_ABC, CD26, CD3, CD195, CD164, CD8/CD8a, HLA-DR, CD194, CD11b, CD38, CD45, CD95, CD223, CD45RA, CD14, CD7, CD123, TIM3, TIGIT, CD19, and FcRL3,
b) CD28, PD-1, CD4, CD3, HLA ABC, TIGIT, CD197, CD27, CD26, CD44, CD194, CD7, CD45RA, CD158k, and CD164,
c) CD3, CD4, CD7, CD8/CD8a, CD26, CD27, CD45, CD45RA and TIGIT, or
d) CD3, CD4, CD7, CD8/CD8a, CD26, CD45RO, TIGIT, CD194/CCR4, CD197/CCR7, and CD279/PD-1.

13. The composition of claim 12, wherein the biomarkers consist of:
a) CD3, CD4, CD7, CD8/CD8a, CD26, CD27, CD45, CD45RA and TIGIT; or
b) CD3, CD4, CD7, CD8/CD8a, CD26, CD45RO, TIGIT, CD194, CD197 and PD-1.

14. The composition of claim 12 or 13, wherein the biomarkers consist of CD3, CD4, CD7, CD8/CD8a, CD26, CD27, CD45, CD45RA and TIGIT.

## Patentansprüche

1. Verfahren zur Bestimmung der Häufigkeit von Sézary-Signaturzellen und/oder Mycosis-fungoides-Zellen in einer Vielzahl von Zellen, wobei das Verfahren die folgenden Schritte umfasst:
i) Bestimmen der Expressionsspiegel von 9 oder mehr Biomarkern in einer Vielzahl von Zellen; und
ii) Bestimmen der Häufigkeit von Sézary-Signaturzellen und/oder Mycosis-fungoides-Zellen in der Vielzahl von Zellen auf der Grundlage der Expressionsspiegel der 9 oder mehr Biomarker;
wobei die Biomarker
a) CD28, CD44, HLA-ABC, CD26, CD3, CD195, CD164, CD8/CD8a, HLA-DR, CD194, CD11b, CD38, CD45, CD95, CD223, CD45RA, CD14, CD7, CD123, TIM3, TIGIT, CD19 und FcRL3,
b) CD28, PD-1, CD4, CD3, HLA-ABC, TIGIT, CD197, CD27, CD26, CD44, CD194, CD7, CD45RA, CD158k und CD164,
c) CD3, CD4, CD7, CD8/CD8a, CD26, CD27, CD45, CD45RA und TIGIT, oder
d) CD3, CD4, CD7, CD8/CD8a, CD26, CD45RO, TIGIT, CD194/CCR4, CD197/CCR7 und CD279/PD-1
umfassen oder daraus bestehen.

2. Verfahren nach Anspruch 1, wobei ein Klassifizierungsalgorithmus verwendet wird, um zwischen einer Sézary-Signaturzelle und einer Nicht-Sézary-Signaturzelle bzw. einer Mycosis-fungoides-Zelle und einer Nicht-Mycosis-fungoides-Zelle zu unterscheiden.

3. Verfahren nach einem der Ansprüche 1 bis 2, wobei ein konvolutionales neurales Netzwerk verwendet wird, um zwischen einer Sézary-Signaturzelle und einer Nicht-Sézary-Signaturzelle bzw. einer Mycosis-fungoides-Zelle und einer Nicht-Mycosis-fungoides-Zelle zu unterscheiden.

4. Verfahren nach einem der Ansprüche 1 bis 3, wobei die Vielzahl von Zellen humane Zellen sind.

5. Verfahren nach einem der Ansprüche 1 bis 4, wobei die Spiegel der Biomarker unter Verwendung eines antikörperbasierten Assays bestimmt werden.

6. Verfahren nach Anspruch 5, wobei der antikörperbasierte Assay ein antikörperbasierter Durchfluss-Zytometrie-Assay oder Massen-Zytometrie-Assay ist.

7. Verfahren nach einem der Ansprüche 1 bis 6, wobei die 9 oder mehr Biomarker ein Panel sind, das neun verschiedene Biomarker umfasst oder daraus besteht, nämlich (i) CD3, (ii) CD4, (iii) CD7, (iv) CD8/CD8a, (v) CD26, (vi) CD27, (vii) CD45, (viii) CD45RA und (ix) TIGIT.

8. Computerimplementiertes Verfahren zur Bestimmung der Häufigkeit von Sézary-Signaturzellen oder Mycosis-fungoides-Zellen, wobei das Verfahren die folgenden Schritte umfasst:
i) Ausführen eines Klassifizierungsalgorithmus für einen Datensatz, der die Expressionsspiegel von 9 oder mehr Biomarkern in einer Vielzahl von Zellen umfasst;
ii) Bestimmen der Häufigkeit von Sézary-Signaturzellen bzw. Mycosis-fungoides-Zellen in der Vielzahl von Zellen, die dem Datensatz zugrunde liegen, auf der Grundlage der Expressionsspiegel der 9 oder mehr Biomarker;
wobei die Biomarker
a) CD28, CD44, HLA_ABC, CD26, CD3, CD195, CD164, CD8/CD8a, HLA-DR, CD194, CD11b, CD38, CD45, CD95, CD223, CD45RA, CD14, CD7, CD123, TIM3, TIGIT, CD19 und FcRL3,
b) CD28, PD-1, CD4, CD3, HLA ABC, TIGIT, CD197, CD27, CD26, CD44, CD194, CD7, CD45RA, CD158k und CD164,
c) CD3, CD4, CD7, CD8/CD8a, CD26, CD27, CD45, CD45RA und TIGIT, oder
d) CD3, CD4, CD7, CD8/CD8a, CD26, CD45RO, TIGIT, CD194/CCR4, CD197/CCR7 und CD279/PD-1
umfassen oder daraus bestehen;
wobei vorzugsweise der Klassifizierungsalgorithmus einen oder eine Kombination aus einem Support-Vektor-Algorithmus, einem konvolutionalen neuralen Netzwerk, einem Baum-basierten Verfahren und einer logistischen Regression umfasst.

9. Verfahren zur Diagnose eines Probanden mit Sézary-Syndrom oder mit Mycosis fungoides, wobei das Verfahren die folgenden Schritte umfasst:
i) Bestimmen der Häufigkeit von Sézary-Signaturzellen bzw. Mycosis-fungoides-Zellen in einer Probe, die von dem Probanden erhalten wurde, unter Verwendung des Verfahrens gemäß einem der Ansprüche 1 bis 7;
ii) Vergleichen der in Schritt (i) bestimmten Häufigkeit von Sézary-Signaturzellen bzw. Mycosis-fungoides-Zellen mit der Häufigkeit von Sézary-Signaturzellen bzw. Mycosis-fungoides-Zellen in einer Probe, die von einem Probanden erhalten wurde, der nicht an dem Sézary-Syndrom bzw. Mycosis fungoides leidet, und/oder mit der Häufigkeit von Sézary-Signaturzellen bzw. Mycosis-fungoides-Zellen in einer Probe, die von einem Probanden mit Sézary-Syndrom bzw. Mycosis fungoides gewonnen wurde; und
iii) Bestimmen, dass ein Proband das Sézary-Syndrom bzw. Mycosis fungoides hat, wenn die in Schritt (ii) bestimmte Häufigkeit von Sézary-Signaturzellen bzw. Mycosis-fungoides-Zellen höher ist als die Häufigkeit von Sézary-Signaturzellen bzw. Mycosis-fungoides-Zellen bei dem Proband, der nicht an dem Sézary-Syndrom bzw. an Mycosis fungoides leidet, und/oder wenn die in Schritt (ii) bestimmte Häufigkeit von Sézary-Signaturzellen bzw. Mycosis-fungoides-Zellen ähnlich oder höher ist als die Häufigkeit von Sézary-Signaturzellen bzw. Mycosis-fungoides-Zellen in der Probe, die von dem Probanden mit Sézary-Syndrom bzw. Mycosis fungoides erhalten wurde.

10. Verfahren nach Anspruch 9,
a) wobei das Bestimmen, dass der Proband an dem Sézary-Syndrom bzw. an Mycosis fungoides leidet, die Verwendung eines Klassifizierungsalgorithmus umfasst;
b) wobei der Klassifizierungsalgorithmus ein konvolutionales neuronales Network und/oder eine logistische Regression umfasst;
c) wobei der Proband ohne Sézary-Syndrom ein gesunder Proband oder ein Proband ist, der an atopischer Dermatitis, unspezifischer Dermatitis, Erythrodermie und/oder Mycosis fungoides leidet;
d) wobei die Probe eine Blutprobe ist, insbesondere wobei die Probe periphere mononukleäre Blutzellen umfasst; und/oder
e) des Weiteren umfassend einen oder mehrere andere diagnostische Tests und/oder zusätzliche Informationen über den Proband, wobei bevorzugt der eine oder die mehreren anderen diagnostischen Tests aus der Gruppe ausgewählt sind, bestehend aus: Ganzkörper-Bildgebungstests, Hautläsionsbiopsien, histopathologischen Tests, Bestimmung des CD4/CD8-Verhältnisses, Zellzählungsanalyse und PCR-Analyse der T-Zell-Rezeptor-Klonalität; und/oder
wobei die zusätzlichen Informationen über den Proband das Alter und/oder klinische Informationen umfassen.

11. Verfahren zur Bestimmung der Ansprechbarkeit auf eine Behandlung des Sézary-Syndroms oder der Mycosis fungoides bei einem Proband, wobei das Verfahren die folgenden Schritte umfasst:
i) Bestimmen der Häufigkeit von Sézary-Signaturzellen bzw. Mycosis-fungoides-Zellen in einer ersten Probe, die von dem Proband erhalten wurde;
ii) Bestimmen der Häufigkeit von Sézary-Signaturzellen bzw. Mycosis-fungoides-Zellen in einer zweiten Probe, die von demselben Proband erhalten wurde, wobei die zweite Probe zu einem späteren Zeitpunkt als die erste Probe erhalten wurde;
iii) Bestimmen der Änderung der Häufigkeit von Sézary-Signaturzellen bzw. Mycosis-fungoides-Zellen zwischen der ersten und der zweiten Probe; und
iv) Bestimmen, dass 7der Proband auf eine Behandlung des Sézary-Syndroms bzw. der Mycosis fungoides anspricht, wenn die Häufigkeit von Sézary-Signaturzellen bzw. Mycosis-fungoides-Zellen in der zweiten Probe geringer ist als in der ersten Probe;
wobei die Häufigkeit von Sézary-Signaturzellen bzw. Mycosis-fungoides-Zellen unter Verwendung des Verfahrens nach einem der Ansprüche 1 bis 7 bestimmt wird, wobei die Probe vorzugsweise eine Blutprobe ist, wobei insbesondere die Probe periphere mononukleäre Blutzellen umfasst.

12. Zusammensetzung, die aus Reagenzien zum Nachweis von Biomarkern zur Diagnose des Sézary-Syndroms oder von Mycosis fungoides besteht, wobei die Biomarker bestehen aus:
a) CD28, CD44, HLA_ABC, CD26, CD3, CD195, CD164, CD8/CD8a, HLA-DR, CD194, CD11b, CD38, CD45, CD95, CD223, CD45RA, CD14, CD7, CD123, TIM3, TIGIT, CD19 und FcRL3,
b) CD28, PD-1, CD4, CD3, HLA ABC, TIGIT, CD197, CD27, CD26, CD44, CD194, CD7, CD45RA, CD158k und CD164,
c) CD3, CD4, CD7, CD8/CD8a, CD26, CD27, CD45, CD45RA und TIGIT, oder
d) CD3, CD4, CD7, CD8/CD8a, CD26, CD45RO, TIGIT, CD194/CCR4, CD197/CCR7 und CD279/PD-1.

13. Zusammensetzung nach Anspruch 12, wobei die Biomarker bestehen aus:
a) CD3, CD4, CD7, CD8/CD8a, CD26, CD27, CD45, CD45RA und TIGIT; oder
b) CD3, CD4, CD7, CD8/CD8a, CD26, CD45RO, TIGIT, CD194, CD197 und PD-1.

14. Zusammensetzung nach Anspruch 12 oder 13, wobei die Biomarker aus CD3, CD4, CD7, CD8/CD8a, CD26, CD27, CD45, CD45RA und TIGIT bestehen.

## Revendications

1. Procédé pour déterminer la fréquence de cellules signature de Sézary et/ou de cellules de mycosis fongoïde dans une pluralité de cellules, le procédé comprenant les étapes de :
i) détermination des niveaux d'expression de 9 biomarqueurs ou plus dans une pluralité de cellules ; et
ii) détermination de la fréquence de cellules signature de Sézary et/ou de cellules de mycosis fongoïde dans la pluralité de cellules sur la base des niveaux d'expression des 9 biomarqueurs ou plus ;
où les biomarqueurs comprennent ou sont constitués par
a) CD28, CD44, HLA-ABC, CD26, CD3, CD195, CD164, CD8/CD8a, HLA-DR, CD194, CD11b, CD38, CD45, CD95, CD223, CD45RA, CD14, CD7, CD123, TIM3, TIGIT, CD19, et FcRL3,
b) CD28, PD-1, CD4, CD3, HLA-ABC, TIGIT, CD197, CD27, CD26, CD44, CD194, CD7, CD45RA, CD158k, et CD164,
c) CD3, CD4, CD7, CD8/CD8a, CD26, CD27, CD45, CD45RA et TIGIT, ou
d) CD3, CD4, CD7, CD8/CD8a, CD26, CD45RO, TIGIT, CD194/CCR4, CD197/CCR7, et CD279/PD-1.

2. Procédé selon l'une quelconque de la revendication 1, dans lequel un algorithme classificateur est utilisé pour distinguer entre une cellule de signature de Sézary et une cellule de signature non de Sézary ou une cellule de mycosis fongoïde et une cellule non de mycosis fongoïde, respectivement.

3. Procédé selon l'une quelconque des revendications 1 à 2, dans lequel un réseau neuronal convolutif est utilisé pour distinguer entre une cellule signature de Sézary et une cellule signature non de Sézary ou une cellule de mycosis fongoïde et une cellule non de mycosis fongoïde, respectivement.

4. Procédé selon l'une quelconque des revendications 1 à 3, dans lequel la pluralité de cellules est des cellules humaines.

5. Procédé selon l'une quelconque des revendications 1 à 4, dans lequel les niveaux des biomarqueurs sont déterminés en utilisant un test à base d'anticorps.

6. Procédé selon la revendication 5, dans lequel le test à base d'anticorps est un test par cytométrie en flux à base d'anticorps ou par cytométrie de masse.

7. Procédé selon l'une quelconque des revendications 1 à 6, dans laquelle les 9 biomarqueurs ou plus sont un panel comprenant ou constitué de neuf biomarqueurs différents, à savoir (i) CD3, (ii) CD4, (iii) CD7, (iv) CD8/CD8a, (v) CD26, (vi) CD27, (vii) CD45, (viii) CD45RA et (ix) TIGIT.

8. Procédé mis en œuvre par ordinateur de détermination de la fréquence de cellules de signature de Sézary ou de cellules de mycosis fongoïde, le procédé comprenant les étapes de :
i) exécution d'un algorithme classificateur sur un ensemble de données comprenant les niveaux d'expression de 9 biomarqueurs ou plus dans une pluralité de cellules ;
ii) détermination de la fréquence des cellules signature de Sézary ou des cellules de mycosis fongoïde, respectivement, dans la pluralité de cellules sous-jacentes à l'ensemble des données basée sur les niveaux d'expression des 9 biomarqueurs ou plus ;
où les biomarqueurs comprennent ou sont constitués par
a) CD28, CD44, HLA_ABC, CD26, CD3, CD195, CD164, CD8/CD8a, HLA-DR, CD194, CD11b, CD38, CD45, CD95, CD223, CD45RA, CD14, CD7, CD123, TIM3, TIGIT, CD19, et FcRL3,
b) CD28, PD-1, CD4, CD3, HLA ABC, TIGIT, CD197, CD27, CD26, CD44, CD194, CD7, CD45RA, CD158k, et CD164,
c) CD3, CD4, CD7, CD8/CD8a, CD26, CD27, CD45, CD45RA et TIGIT, ou
d) CD3, CD4, CD7, CD8/CD8a, CD26, CD45RO, TIGIT, CD194/CCR4, CD197/CCR7, et CD279/PD-1 ;
de préférence, dans lequel l'algorithme classificateur comprend un ou une combinaison d'un algorithme à vecteurs de support, d'un réseau de neurones convolutif, d'un procédé à base d'arbre, d'une régression logistique.

9. Procédé pour diagnostiquer un sujet comme étant atteint d'un syndrome de Sézary ou comme étant atteint de mycosis fongoïde, le procédé comprenant les étapes de :
i) détermination de la fréquence de cellules signature de Sézary ou de cellules de mycosis fongoïde, respectivement, dans un échantillon obtenu à partir du sujet par le procédé selon l'une quelconque des revendications 1 à 7 ;
ii) comparaison de la fréquence des cellules signature de Sézary ou des cellules de mycosis fongoïde, respectivement, déterminée à l'étape (i), à la fréquence des cellules signature de Sézary ou des cellules de mycosis fongoïde, respectivement, dans un échantillon obtenu à partir d'un sujet ne souffrant pas de syndrome de Sézary ou de mycosis fongoïde, respectivement, et/ou à la fréquence des cellules signature de Sézary ou des cellules de mycosis fongoïde, respectivement, dans un échantillon obtenu à partir d'un sujet souffrant de syndrome de Sézary ou de mycosis fongoïde ; et
iii) détermination d'un sujet comme ayant le syndrome de Sézary ou de mycosis fongoïde, respectivement, si la fréquence de cellules signature de Sézary ou de cellules de mycosis fongoïde, respectivement, déterminées à l'étape (ii) est supérieure à la fréquence de cellules signature de Sézary ou de cellules de mycosis fongoïde, respectivement, pour le sujet ne souffrant pas de syndrome de Sézary ou de mycosis fongoïde, respectivement, et/ou si la fréquence des cellules signature de Sézary ou des cellules de mycosis fongoïde, respectivement, déterminée à l'étape (ii) est similaire ou supérieure à la fréquence des cellules signature de Sézary ou des cellules de mycosis fongoïde, respectivement, dans l'échantillon obtenu à partir du sujet souffrant de syndrome de Sézary ou de mycosis fongoïde, respectivement.

10. Procédé selon la revendication 9,
a) dans lequel la détermination du sujet comme étant atteint du syndrome de Sézary ou de mycosis fongoïde, respectivement, comprend l'utilisation d'un algorithme classificateur ;
b) dans lequel l'algorithme classificateur comprend un réseau de neurones convolutif et/ou une régression logistique ;
c) dans lequel le sujet sans syndrome de Sézary est un sujet sain ou un sujet souffrant de dermatite atopique, dermatite non spécifique, érythrodermie et/ou mycose fongoïde ;
d) dans lequel l'échantillon est un échantillon de sang, en particulier, dans lequel l'échantillon comprend des cellules mononucléaires de sang périphérique ; et/ou
e) comprenant en outre un ou plusieurs autres tests diagnostiques et/ou des informations supplémentaires sur le sujet, de préférence dans lequel le ou les autres tests diagnostiques est/sont choisi(s) dans le groupe constitué par : tests d'imagerie corporelle entière, biopsies de lésion cutanée, tests histopathologiques, détermination du rapport CD4/CD8, analyse du comptage cellulaire et analyse PCR de la clonalité des récepteurs de lymphocytes T ; et/ou
dans lequel les informations supplémentaires sur le sujet comprennent l'âge et/ou des informations cliniques.

11. Procédé pour déterminer la sensibilité au traitement du syndrome de Sézary ou de mycosis fongoïde chez un sujet, le procédé comprenant les étapes de :
i) détermination de la fréquence de cellules signature de Sézary ou de cellules de mycosis fongoïde, respectivement, dans un premier échantillon obtenu à partir dudit sujet ;
ii) détermination de la fréquence de cellules signature de Sézary ou de cellules de mycosis fongoïde, respectivement, dans un second échantillon obtenu à partir du même sujet, dans lequel le second échantillon a été obtenu à un moment ultérieur à celui du premier échantillon ;
iii) détermination du changement de fréquence des cellules signature de Sézary ou des cellules de mycosis fongoïde, respectivement, entre le premier et le second échantillon ; et
iv) détermination si le sujet est sensible au traitement du syndrome de Sézary ou de mycosis fongoïde, respectivement, si la fréquence des cellules signature de Sézary ou des cellules de mycosis fongoïde, respectivement, est plus faible dans le deuxième échantillon que dans le premier échantillon ;
dans lequel la fréquence des cellules de signature de Sézary ou des cellules de mycosis fongoïde, respectivement, est déterminée en utilisant le procédé selon l'une quelconque des revendications 1 à 7, de préférence dans lequel l'échantillon est un échantillon de sang, en particulier, dans lequel l'échantillon comprend des cellules mononucléaires de sang périphérique.

12. Composition constituée de réactifs pour la détection de biomarqueurs pour le diagnostic du syndrome de Sézary ou de mycosis fongoïde, les biomarqueurs étant constitués par :
a) CD28, CD44, HLA_ABC, CD26, CD3, CD195, CD164, CD8/CD8a, HLA-DR, CD194, CD11b, CD38, CD45, CD95, CD223, CD45RA, CD14, CD7, CD123, TIM3, TIGIT, CD19, et FcRL3,
b) CD28, PD-1, CD4, CD3, HLA ABC, TIGIT, CD197, CD27, CD26, CD44, CD194, CD7, CD45RA, CD158k, et CD164,
c) CD3, CD4, CD7, CD8/CD8a, CD26, CD27, CD45, CD45RA et TIGIT, ou
d) CD3, CD4, CD7, CD8/CD8a, CD26, CD45RO, TIGIT, CD194/CCR4, CD197/CCR7, et CD279/PD-1.

13. Composition selon la revendication 12, dans laquelle les biomarqueurs sont constitués par :
a) CD3, CD4, CD7, CD8/CD8a, CD26, CD27, CD45, CD45RA et TIGIT ; ou
b) CD3, CD4, CD7, CD8/CD8a, CD26, CD45RO, TIGIT, CD194, CD197 et PD-1.

14. Composition selon la revendication 12 ou 13, dans laquelle les biomarqueurs sont constitués par CD3, CD4, CD7, CD8/CD8a, CD26, CD27, CD45, CD45RA et TIGIT.
